# EUROPÄISCHE PATENTSCHRIFT

(11) **EP 0 816 852 B1**
(45) Veröffentlichungstag und Bekanntmachung des Hinweises auf die Patenterteilung: **13.10.2004**
(21) Anmeldenummer: 97890118.9
(22) Anmeldetag: 02.07.1997
(51) Int. Cl.: G01N 33/68, G01N 33/566, G01N 33/543, G01N 33/535, A61L 27/00, C07K 1/22, A61L 15/32

(54) **Verfahren zur Bestimmung einer Kollagen-bindenden Substanz, insbesondere der Aktivität eines Adhäsionsproteins**
Process for measuring a collagen-binding substance, especially of the activity of an adhesion-protein
Procédé de détermination d'une substance liant le collagène, en particulier de l'activité d'une protéine d'adhésion

(30) Priorität: 04.07.1996 AT 119096; 18.12.1996 AT 221796
(43) Veröffentlichungstag der Anmeldung: 07.01.1998
(73) Patentinhaber: Baxter Aktiengesellschaft, 1221 Wien (AT)
(72) Erfinder: Siekmann, Jürgen, Dr., 1210 Wien (AT); Turecek, Peter, Dr., 3400 Klosterneuburg (AT); Schwarz, Hans-Peter, Prof., 1180 Wien (AT); Eibl, Johann, Dr., 1180 Wien (AT); Fischer, Bernhard, Doz, 1120 Wien (AT); Mitterer, Artur, Dr., 2304 Mannsdorf 116 (AT); Dorner, Friedrich, Prof., 1230 Wien (AT)
(74) Vertreter: Alge, Daniel, Mag. Dr. rer.nat.

(56) Entgegenhaltungen:
- Chem. abstr., Band 123, Nr. 3, 17. Juli 1995 (Columbus, Ohio, USA), Seite 359, the abstract No. 28257w, THOSMAS, K.B. et al. "A simple test for the determination of the function of von Willebrand factor: collagen-binding aktivity", Haemostaselogie (Stuttgart) 1994, 14(3), 133-9.
- Chem. abstr., Band 118, Nr. 12, 22. März 1993 (Columbus, Ohio, USA), Seite 544, the abstract No. 109608k, OKADA, T. et al. "In vitro and in vivo digestion of collagen covalently immobilized onto the silicone surface", J. Biomed. Mater. Res. 1992, 26(12), 1569-81.
- Chem. abstr., Band 117, Nr. 3, 20. Juli 1992 (Columbus, Ohio, USA), Seite 335, the abstract No. 22775t, FAVALORO, E.J. et al. "Development of a simple collagen based ELISA assay aids in the diagnosis of, and permits sensitive discrimination between type I and type II, von Willebrand's disease", Blood Coagulation Fibrinolysis 1991, 2(2), 285-91.
- Chem. abstr., Band 116, Nr. 10, 09. März 1992 (Columbus, Ohio, USA), Seite 502, the abstract No. 91309k, KOBAYASHI, H. et al. "Covalent immobilization of proteins onto the surface of Poly(vinylalcohol)hydrogel", Biomaterials 1991, 12(8), 747-51.
- Chem. abstr., Band 112, Nr. 25, 18. Juni 1990 (Columbus, Ohio, USA) Seite 309, the abstract No. 232155p, GILCHRIST, M. et al. "Rapid diagnosis of von Willebrand's disease using ELISA technology", Thromb. Res. 1990, 57(4), 659-64.
- Chem. abstr., Band 105, Nr. 15, 13. Oktober 1986 (Columbus, Ohio, USA) Seite 338, the abstract No. 130139z, KOETTGEN, E. et al. "Functional analysis of plasma fibronectin with special consideration of binding interferences", J. Clin. Chem. Clin. Biochem. 1986, 24(8), 541-9.
- Chem. abstr., Band 91, Nr. 11, 10. September 1979 (Columbus, Ohio, USA), Seite 361, the abstract No. 86578h, ENGVALL, E. et al. "Principles of ELISA and recent applications to the study of molecular interactions", Lab. Res. Methods Biol. Med. 1979, 3(Immunoassays Clin. Lab.) 89-97.
- THOMAS K.B ET AL: 'EIN EINFACHER TEST FUER DIE BESTIMMUNG DER FUNKTION DES VON-WILLEBRAND-FAKTORS: DIE KOLLAGENBINDUNGSAKTIVITAET' HAEMOSTASELOGIE Bd. 14, Nr. 3, 1994, STUTTGART, Seiten 133 - 139, XP000916123
- OKADA ET AL J.BIOMED.MAT.RES. Bd. 26, 1992, Seiten 747 - 751
- KOBAYASHI H. ET AL: 'COVALENT IMMOBILIZATION OF PROTEINS ON TO THE SURFACE OF POLY(VINYL ALCOHOL) HYDROGEL' BIOMATERIALS Bd. 12, Nr. 8, 1991, Seiten 747 - 751, XP000916131
- WILKINSON M.J. ET AL: 'NONRADIOACTIVE ASSAY FOR TYPE IV COLLAGEN DEGRADATION' ANAL.BIOCHEM. Bd. 185, 1990, Seiten 294 - 296, XP000917296

## Beschreibung

Die Erfindung betrifft ein Verfahren zur Bestimmung einer Kollagen-bindenden Substanz, insbesondere zur Bestimmung der Aktivität von Adhäsionsproteinen sowie Vorrichtungen zur Durchführung dieses Verfahrens.

Die Blutgerinnung wird durch eine Reihe von aufeinanderfolgenden Reaktionen verschiedener Proteine und Enzyme ausgelöst. Durch einen Mangel oder eine Fehlfunktion eines Blutgerinnungsfaktors wird die Bildung des Wundverschlusses verhindert, die Folge sind Blutungen, die bis zum Tod führen können. Ein solcher Fall liegt z.B. bei der von Willebrand-Krankheit vor, die durch das Fehlen oder eine Störung des Adhäsionsfaktors vWF gekennzeichnet ist.

Der von Willebrand-Faktor ist ein multimeres Plasmaprotein, bestehend aus Untereinheiten mit Molekulargewichten von 225,000 D. Zwei Untereinheiten bilden ein über Schwefelbrücken verbundenes Dimer mit einem Molekulargewicht von 450,000 D. Durch kovalente Verknüpfung werden aus den vWF-Dimeren Polymere mit Molekulargewichten von 1 Million und mehr gebildet. Bei der Blutgerinnung erfüllt der vWF zwei wesentliche Aufgaben. 1. Der vWF bindet den Gerinnungsfaktor VIII (FVIII), und 2. verfügt der vWF über adhäsive Eigenschaften, die die Aggregation von Blutplättchen und deren Bindung an das Subendothelium der Gefäßwand, via Kollagen, vermitteln. Es wird allgemein davon ausgegangen, daß die Funktionalität des vWF wesentlich von seiner Multimerstruktur abhängt. So wird angenommen, daß nur die hochmolekularen vWF-Polymere die hämostatisch aktiven Moleküle darstellen.

Dem von Willebrand-Faktor (vWF) kommt eine besondere Bedeutung in der physiologischen Hämostase zu. Neben seiner Funktion als Carrier-Protein für Faktor VIII ist dieses Plasmaprotein vor allem für die Adhäsion von Thrombozyten an das geschädigte Endothel/Subendothel sowie für die Aggregation der Thrombozyten unter Scherstreßbedingungen notwendig. Im ersten Schritt der primären Hämostase, der Adhäsion, fungiert der vWF als ein Bindeglied zwischen spezifischen Rezeptoren der Thrombozytenoberfläche, wie gpIb-, gpIIb/IIIa-Komplex, und Komponenten des Endothels, beispielsweise Kollagen.

In der Folge kommt es zu einer Formveränderung der Thrombozyten, der Sekretion von Inhaltsstoffen sowie schließlich zur Bildung von Thrombozyten-Aggregaten, die auch durch vWF mediiert wird.

Die verschiedenen Erscheinungsbilder der von Willebrand-Krankheit (vWD) sind durch den Verlust der hochmolekularen vWF-Polymere gekennzeichnet, offenbar Ergebnis der verringerten adhäsiven Eigenschaften des vWF, was zur Blutungsneigung und Verlängerung der Blutungszeit führt. Aufgrund des vWF-Musters findet eine Klassifizierung der von Willebrand-Krankheit in 3 Haupttypen sowie mehrere Subtypen statt. Im Blut von Typ I-Patienten wird eine quantitative Reduktion aller vWF-Multimeren, verbunden mit einem reduziertem vWF-Antigengehalt (vWF:Ag) und vWF Ristocetin-Cofaktor-Aktivität (vWF:RCo) gefunden, während sich der Typ III durch ein komplettes Fehlen des vWF auszeichnet. Der Typ II ist durch eine qualitative Abnormität und die Abwesenheit der mittelgroßen und großen vWF-Multimere sowie stark reduzierte vWF:RCo-Aktivität charakterisiert. Da die hochmolekularen vWF-Multimeren die ausgeprägtesten adhäsiven Eigenschaften besitzen, führt diese qualitative Abnormität des vWF ebenfalls zur Blutungsneigung und Verlängerung der Blutungszeit.

Zur Differentialdiagnose der vWD werden eine Reihe von Untersuchungen durchgeführt. Gebräuchlich sind der Hautblutungstest und funktionelle Tests, die die adhäsiven Eigenschaften des vWF untersuchen. Bei der Charakterisierung der adhäsiven Eigenschaften des vWF wird üblicherweise die Blutplättchenaggregation untersucht. Dazu wird vWF mit Formalin-stabilisierten Blutplättchen und dem unphysiologischen Antibiotikum Ristocetin gemischt, und die Zeitdauer und Intensität der Plättchenaggregation gemessen. Dieses Meßsystem (Ristocetin-abhängige Plättchenaggregation) spiegelt jedoch nicht die physiologische Situation und Funktion des vWF wider.

Grundprinzip dieser Bestimmung der Ristocetin-Cofaktor-Aktivität ist die Induzierung der Agglutination von fixierten oder frisch präparierten gewaschenen Thrombozyten in Gegenwart von vWF durch das Antibiotikum Ristocetin (Weiss et al., J.Clin.Invest. 54 (1973), 2708-2716). Die Agglutination ist dabei von der Menge des vorhandenen aktiven vWF abhängig. Dessen Konzentration läßt sich aus einer photometrisch aufgenommenen Agglutinationskurve mit Hilfe eines Aggregometers im Vergleich zu einem geeigneten Standard bestimmen. Problematisch ist jedoch bei diesem Test, daß die RCoF-Werte je nach Thrombozyten-Präparation variieren können. Außerdem ist dieser Test relativ unempfindlich, die Nachweisgrenze für den aktiven vWF liegt bei ungefähr 1 µg/ml. Darüberhinaus ist diese Funktion des vWF zur Aggregation von Thrombozyten in Gegenwart von Ristocetin eine unphysiologische und möglicherweise ein ungeeignetes Modell der primären hämostatischen Aktivität. Dieses Modell wird lediglich zur Bestimmung des vWF herangezogen und ist auf weitere Adhäsionsproteine nicht übertragbar.

Das Prinzip der Agglutinometrie wird auch zur Bestimmung von Antigen/Antikörper-Wechselwirkungen verwendet. So lassen sich Antikörper aufgrund physikalischer Adsorption (Crane, Clin.Chem. 27 (1981), 697-700) oder auch durch Bildung einer kovalenten Bindung (US-PS 4 480 042; US-PS 4 210 723; Thakkar et al., Clin. Chem. 37 (1991), 1248-1251) an Mikropartikel aus Kunstharzen immobilisieren. Die Größe und die optischen Eigenschaften der Teilchen sind dann abhängig von der Konzentration des betreffenden Antigens/Antikörpers, wodurch die Bestimmung der Konzentration des Antigens bzw. Antikörpers, beispielsweise mit Hilfe eines Aggregometers, nephelometrisch oder turbidimetrisch möglich ist.

Ein anderes System zur Erfassung der adhäsiven Eigenschaften des vWF besteht in der Bestimmung der Bindung des vWF zu Kollagen. Kollagen stellt eine Grundsubstanz des subendothelialen Gewebes dar. Das Kollagen Typ III ist der Hauptbestandteil der extrazellulären Matrix der Blutgefäße. Er verleiht diesen eine hohe mechanische Festigkeit bei gleichzeitiger Elastizität. Das Kollagen ist in intakten Blutgefäßen durch einen Monolayer von einer das Blutgefäß luminal ausgekleideten Endothelzellschicht von den zellulären und löslichen Blutkomponenten isoliert. Bei einer Gefäßverletzung wird das Endothel lokal zerstört, sodaß nun direkter Kontakt zwischen Kollagen und Blut erfolgen kann. An das exponierte Kollagen bindet sich das Plasmaprotein vWF, das wiederum durch den vWF-Rezeptor (GP Ib/IX) und den aktivierten Fibrinogen-Rezeptor (GPIIb/IIIa) der Blutplättchen gebunden werden kann. Dadurch kommt es zur vWF-vermittelten Adhäsion der Blutplättchen an das freiliegende Subendothel und somit zum ersten, labilen Wundverschluß. Die vorwiegend in tieferen Gefäßschichten lokalisierten Kollagene des Typs I und Typs VI sind ebenfalls an der Bindung des vWF an das Subendothel beteiligt. Die Bestimmung einer vWF-Kollagen-Bindung entspricht dabei der physiologischen Funktion des vWF.

Über die exakte Bindungsstelle von vWF an Kollagen besteht noch Unklarheit. Es gibt jedoch Hinweise, daß die A1 und A3 Domäne von vWF-Bindungsstellen für Kollagen besitzen (Ruggeri et al. (1993), FASEB J. 7:308-316). Cruz et al. ((1995), J. Biol. Chem. 270:10822-10827) geben als wahrscheinlichste Bindungsstellen die Aminosäuresequenz zwischen Glu¹⁰¹⁸ und Arg¹¹¹⁴ in der A3-Domäne an. Die Bindung des vWF an den vWF-Rezeptor GP Ib der Blutplättchen wiederum erfolgt durch Bindungsstellen innerhalb des Loops Cys⁵⁰⁹-Cys⁶⁹⁵ von vWF (Ruggeri et al. (1992), Thromb. Haemost. 67:594-599).

In vergleichenden Untersuchungen konnte eine gute Übereinstimmung der Kollagen-Bindungsdaten mit den Ergebnissen der Ristocetin-abhängigen Plättchenaggregation gezeigt werden (Brown et al. (1986), Thromb. Res. 43:303-311, Favaloro et al. (1994), Am. J. Hematol. 45:205-211, Thomas et al. (1994), Hämostaseologie 14: 133-139) und es wurde vorgeschlagen, den vWF:RCo-Test gegen einen Kollagen-ELISA zur Bestimmung der vWF-funktionellen Aktivität in vitro auszutauschen. Aufgrund der hohen Affinität der hochmolekularen vWF-Multimeren für Kollagen (Favaloro et al. (1995), Am. J. Clin. Pathol. 104:264-271) eignet sich dieser Test besonders gut zur Differenzierung von vWD Typ II- und Typ III-Patienten und Gesunden. Die wesentlichen Vorteile des Kollagenbindungstests im Vergleich zum Ristocetin-Bindungstest liegen aber nicht nur in der besseren physiologischen Relevanz, sondern auch im geringeren apparativen Aufwand und der einfacheren und schnelleren Durchführung. Neben einer Anwendung des Kollagenbindungstest bei der Klassifizierung der von Willebrand-Krankheit wurde vorgeschlagen, diesen Test auch zum Monitoring von Patienten mit vWD während und nach der Behandlung mit Desmopressin, für die Qualitätssicherung des vWF in Blutgerinnungsfaktor VIII-Präparaten, sowie zur Beobachtung von Patienten mit anderen Erkrankungen, wie beispielsweise akuter lymphoblastischer Leukämie, hämolytisch-urämischem Syndrom (Moschkowitz-Syndrom) oder autoimmun-thrombotischer Purpura anzuwenden (Favaloro et al. (1994), Am. J. Hematol. 45:205-211, Brown et al. (1986), Thromb. Res. 43: 303-311, Thomas et al. (1994), Hämostaseologie 14:133-139) oder ein Einsatz des Kollagenbindungstest zum Nachweis und zur Klassifizierung der Kollagen-spezifischen Autoantikörper bei rheumatoider Arthritis und bei systemischem Lupus erythematosus.

Zur Bestimmung der vWF-Kollagen-Bindung wurden bisher solche Systeme vorgeschlagen, in denen Kollagen mittels unspezifischer hydrophober Wechselwirkungen an synthetische Oberflächen, z.B. Mikrotitrationsplatten, angelagert wurde. Aufgrund seiner Funktion als fibrilläres Protein ist Kollagen unter physiologischen Bedingungen nicht löslich. Im gereinigten Zustand kann es nur durch starke Säuren oder Salze in Lösung gebracht werden. Insbesondere im neutralen und basischen pH-Bereich wird gelöstes Kollagen wieder unlöslich und fällt aus der Lösung aus. Bei der Verwendung von synthetischen Oberflächen, z.B. Mikrotitrationsplatten, zur Immobilisierung von Proteinen müssen neutrale bis basische Pufferbedingungen erzeugt werden, um Proteine über unspezifische Bindungen an diese Oberflächen zu binden. Durch die Unlöslichkeit von Kollagen, insbesondere unter basischen Bedingungen, ist dieser Weg zur Bindung von Kollagen an synthetische Oberflächen nicht erfolgreich. Um dennoch Kollagen über einen unspezifischen Mechanismus an synthetische Oberflächen zu binden, war es bisher notwendig, sehr hohe Kollagenkonzentrationen einzusetzen, um zumindest geringe Bindungen zur synthetischen Oberfläche herzustellen.

Brown et al. (1986, Thromb. Res. 43:303-311) inkubierten 3,3 µg Kollagen/Mikrotiterplattenvertiefung (=33 mg/ml) über Nacht, wobei bei der Verdünnung darauf geachtet wurde, daß Kollagen nicht ausfällt. Um die Dauer der Bindung von Kollagen an synthetische Oberflächen zu verkürzen, verwendete Bockenstedt et al. (1986, J. Clin. Invest. 78:551-556) 187 µg Kollagen/Mikrotiterplattenvertiefung (1,87 mg/ml, in 0,05 M Essigsäure gelöst) und eine Inkubationsdauer von 1,5 Stunden zur Herstellung einer synthetischen Kollagenoberfläche. Unter diesen Bedingungen wurden maximal 10 µg Kollagen/Mikrotiterplattenvertiefung, also 1/18 der eingesetzten Menge, gebunden. Die Bindungskapazität betrug dabei 7,5 ng vWF/µg Kollagen. Favaloro et al. (1991, Blood Coagul. Fibrinol. 2: 285-291) benutzen ein spezielles HORM-Kollagen und eine isotonische Glucoselösung mit pH 2,8 als Bindungslösung und eine Inkubationszeit von mindestens 48 Stunden, um eine Bindung von 10 µg Kollagen/Mikrotiterplattenvertiefung (=50 µg/ml, in einer isotonischen Glucose-Lösung mit pH 2,8) an die Oberfläche zu erzielen. van Genderen et al. (1994, Blood 84: 3378-3384) benutzen 20 µg Kollagen/Mikrotiterplattenvertiefung (=200 µg/ml, gelöst in 20 mmol Essigsäure) mit einer Kontaktdauer von mehr als 12 Stunden zur Darstellung einer synthetischen Kollagenoberfläche. 5 µg Kollagen/Mikrotiterplattenvertiefung (=50 µg/ml, in Essigsäure gelöst) und Bindungszeiten von mehr als 12 Stunden wurden von Niesvizky et al. (1994, J. Lab. Clin. Med., 123: 137-142) verwendet.

Die angeführten Verfahren zur passiven Immobilisierung von Kollagen benötigen entweder hohe Kollagenkonzentrationen und/oder eine lange Kontaktzeit, um synthetische Kollagenoberflächen darzustellen. Durch die Länge der Inkubationszeit von Kollagen mit der synthetischen Oberfläche ist außerdem davon auszugehen, daß ein Großteil des Kollagens frühzeitig aus der Inkubationslösung ausfällt und somit nicht für die Bindung an der Oberfläche zur Verfügung steht.

Thomas et al. (1994, Hämostaseologie 14: 133-139) beschreiben eine vWF-Kollagenbindungsaktivitäts-(CBA)-ELISA, der für die Handhabung im Routinelabor geeignet und standardisierbar ist, und bei dem diesselben Probenverdünnungen, Puffer und Instrumente wie bei einem ELISA für vWF:Ag benutzt werden können. Sie konnten zeigen, daß die mit dem vWF:CBA-ELISA ermittelten Daten gut mit den vWF:RCo-Werten korrelieren. Die durch unspezifische Wechselwirkung mit 3 µg Kollagen/Mikrotiterplattenvertiefung beschichteten Platten konnten bei 4°C jedoch nur 48 h gelagert werden.

Favarolo et al. (Blood Coagulation and Fibrinolysis 2 (1991), 285-291) konnten mit einem CBA-System bei Patienten mit von Willebrand-Jürgens-Syndrom zwischen den Typen I und II differenzieren.

Zur Bestimmung der Kollagenase-Aktivität wurde von Wilkinson et al. (1990, Anal. Biochem. 185:294-296) ein alternatives Verfahren zur Kopplung von Kollagen an Mikrotiterplatten beschrieben. Kollagen wurde mittels Biotin-N-Hydroxysuccinimid-Ester im Proteinanteil biotinyliert und an Avidin-modifizierte Mikrotiter platten durch hochaffine Bindung immobilisiert. Durch dieses Vorgehen konnte bei einer Konzentration von 5 µg biotinyliertem Kollagen/Mikrotiterplattenvertiefung (50 µg/ml) die Beschichtungszeit auf 1 Stunde bei Raumtemperatur reduziert werden. Die so erhaltene Kollagenoberfläche wurde mit Kollagenase inkubiert und über die freigesetzten, biotinylierten Kollagenbruchstücke die Kollagenase-Aktivität bestimmt. Die Biotinylierung führt dabei zur Bindung an die primären Aminosäuren des Kollagens, wodurch ein derart modifiziertes Molekül möglicherweise in seinen Bindungseigenschaften und seiner Affinität zu funktionell Kollagen-bindenden Proteinen beeinträchtigt wird. Ob ein im Proteinteil verändertes Kollagen noch in der Lage ist, an ein adhäsives Protein zu binden, ist daher nicht vorhersagbar.

Die EP 0 713 707 beschreibt eine Kollagen-Polymer-Matrix, bei der Kollagen über primäre Aminogruppen, insbesondere Lysinreste im Proteinteil kovalent an ein aktiviertes, synthetisches Polymer gebunden wird. Die nicht an das Polymer gebundenen primären Aminogruppen des Kollagens werden zur weiteren Kopplung eines biologisch aktiven Agens benutzt. Die Kollagen-Polymer-Matrix wird zur Herstellung von Implantaten in der Medizin verwendet.

Siekmann et al. (1995, Thromb. Haemost. 73:1160) beschreiben mehrere Varianten eines vWF-Kollagenbindungs-ELISAs und stellten fest, daß abhängig von der Art der Kopplung des Kollagens an die Matrix (unspezifische Bindung oder Bindung über polyklonale Kollagen-Antikörper) das Detektionslimit zwischen 15 und 50 ng vWF/ml Ausgangslösung liegt. Die Variante der Bindung von Kollagen über polyklonale Antikörper an die Matrix benötigten dabei eine geringere Kollagenkonzentration und lieferte eine höhere Sensitivität als eine passive Immobilisierung.

Santoro et al. (1977, J. Clin.Invest. 60:1054-1060) charakterisieren Periodat-oxidiertes Kollagen und zeigen, daß der Kohlenhydratanteil des Kollagens nicht für die Plättchenaggregation notwendig ist.

Shadle et al. (1982, J. Cell Biology 95: 361-365) koppelten Kollagen kovalent an Plastikträger und modifizierten die Oligo saccharide des Kollagens, um dem Einfluß des Kohlenhydratanteils des Kollagens auf die Plättchenadhäsion zu untersuchen.

Ein Problem der Verwendung eines Kollagenbindungstestsystems zur Bestimmung der Affinität eines Adhäsionsproteins an Kollagen stellt die Bereitsstellung einer einheitlich mit Kollagen beschichteten Oberfläche dar. Ein weiteres Problem bei der Verwendung von mit herkömmlichen Methoden beschichteten Kollagen-Trägern ist ihre kurze Lagerfähigkeit, da das Kollagen auch im sauren pH-Bereich nach einiger Zeit ausfällt und sich wieder vom Träger löst oder auch aggregiert. Dadurch wird die Sensitivität und die Reproduzierbarkeit des Testsystems stark beeinträchtigt. Für einen ausreichend guten CBA-Test wurden daher bisher die beschichteten Träger erst kurz vor Gebrauch hergestellt und anschließend unmittelbar verwendet. Für den Einsatz in der Routine oder gar die kommerzielle Produktion von Testsystemen zur Bestimmung der CBA eines Kollagen-bindenden (Plasma)-Proteins sind die im Stand der Technik beschriebenen beschichteten Träger daher nur begrenzt einsetzbar.

Weiters sind die bekannten vWF-CBA-Systeme schlecht reproduzierbar und zeigen oft starke Schwankungen bei der Korrelation gegenüber den RCoF- oder den immunologischen vWF-Antigen-Bestimmungen. Vor der Durchführung eines CBA mußte auch die Mikrotiterplatte frisch mit Kollagen beschichtet werden, um ausreichend Bindungssubstrat in seiner nativen Form zur Verfügung zu stellen. Diese Vorgangsweise erfordert viel Geschick und ist für Routineuntersuchungen, vor allem für die Kontrolle von vWF-Präparaten ungeeignet.

Der vorliegenden Erfindung liegt die Aufgabenstellung zugrunde, verbesserte quantitative Bestimmungsverfahren für die Aktivität eines von Willebrand-Faktors und/oder von weiteren Kollagen-bindenden Substanzen, insbesondere Adhäsionsproteinen zu entwickeln, welche ein einfaches aber exaktes Bestimmen der Proteine ermöglichen. Das neue Bestimmungsverfahren soll insbesondere zur Kontrolle der Qualität von hochkonzentrierten vWF-Präparaten geeignet sein.

Eine weitere Aufgabe der Erfindung besteht darin, eine stabile Kollagen-Oberfläche zur Verfügung zu stellen, die die oben beschriebenen Nachteile nicht aufweist, einfach herzustellen und zu standardisieren ist. Ein weiteres Ziel der Erfindung ist ein verbessertes Verfahren zur quantitativen Bestimmung von Kollagen-bindenden Substanzen, insbesondere von Proteinen mit adhäsiven Eigenschaften, sowie zur Bestimmung der Kollagen-Bindungskapazität dieser Substanzen.

Diese Aufgaben werden erfindungsgemäß gelöst durch ein Verfahren zur Bestimmung einer Kollagen-bindenden Substanz, insbesondere zur Bestimmung der Aktivität von Adhäsionsproteinen, in einer Probe, welches dadurch gekennzeichnet ist, dass Kollagen über eine Modifikation im Kohlenhydratanteil an eine feste Phase kovalent fixiert, die Kollagen-bindende Substanz aus der Probe an das Kollagen gebunden wird und die gebundene Kollagen-bindende Substanz bestimmt wird.

Avides Kollagen, also Kollagen, das mindestens eine Modifikation im Kohlenhydratanteil aufweist, besitzt eine außerordentlich hohe Bindungskapazität zur Adhäsion von Kollagen-bindenden Substanzen, insbesondere von Proteinen, ist gekennzeichnet durch eine Vielzahl von zugänglichen Bindungsstellen für die zu bestimmende Substanz. Dabei sind vor allem die Bindungsstellen für die Avidität bzw. Bindungsfunktion des Kollagens verantwortlich, die die rasche Adhäsion des Bindungssubstrates ermöglichen mit einer ausreichenden Adhäsionskraft. Die Kriterien zur Auswahl eines geeigneten Kollagens sind vielfältig. Einerseits kann von einem Material ausgegangen werden, welches bekanntermaßen die geeigneten Bindungsfähigkeiten besitzt und gegebenenfalls zur Erhöhung der Stabilität prozessiert wird. Die Prozessierung kann aber auch mit dem Ziel der Vergrößerung bzw. Homogenität der Oberfläche des immobilisierten Kollagens erfolgen, was Voraussetzung für das reproduzierbare Ausmaß der Adsorption eines Bindungspartners ist.

Die Avidität eines Kollagens wird bevorzugterweise im bereits immobilisierten Zustand, also gebunden an einem Träger, bestimmt mit einer Standardpräparation, welche üblicherweise für die Kalibrierung von entsprechenden ELISA-Systemen verwendet wird. Die Bestimmung der Avidität wird dabei zweckmäßigerweise unter gleichen Bedingungen durchgeführt, wie bei der Bestimmung der Kollagen-bindenden Substanz.

Im Gegensatz zu den CBA des Standes der Technik, bei denen das "Coating" einer festen Phase mit Kollagen üblich ist, ist es erstmals durch das erfindungsgemäße Testsystem möglich, ein reproduzierbares, lagerstabiles und damit kommerziell einsetzbares Verfahren bzw. Mittel zur Verfügung zu stellen, welches auch für Routineuntersuchungen einsetzbar ist.

Es war nicht vorauszusehen, daß ein Kollagen in chemisch fixierter Form noch als Rezeptor für eine Kollagen-bindende Substanz, insbesondere für ein Adhäsionsprotein, eingesetzt werden kann. Um nämlich die irreversible Änderung bzw. Denaturierung des Kollagens zu vermeiden, was eine stark reduzierte Aktivität und Avidität zur Folge hätte, wurde bei den CBA des Standes der Technik darauf geachtet, die Nativität und Struktur des Kollagens nicht zu beeinträchtigen und in schonender Weise eine Beschichtung vorzunehmen, die allein auf nicht-kovalente Wechselwirkungen beruht.

Die von Brown et al. und Favaloro et al. für die CBA verwendeten Kollagenlösungen waren äußerst hochkonzentriert (33 bzw. 50 oder 100 µg/ml). Reihenversuche hatten gezeigt, daß eine Mindestkonzentration von 50 µg/ml zur Vorbereitung des Kollagenimmobili-sates erforderlich ist. Diese großen Mengen an Kollagen waren vor allem deshalb notwendig, um eine zur Detektion ausreichende Menge an vWF zu binden, da das verwendete Kollagen an sich nicht hinsichtlich dessen Avidität ausgewählt wurde.

Dieses Kollagen behält auch nach dessen Prozessierung und Desintegration durch mechanische oder proteolytische Behandlung die Affinität zur Kollagen-bindenden Substanz, welche Affinität durch diese Behandlung noch zusätzlich gesteigert werden kann.

Unter avidem Kollagen wird erfindungsgemäß Kollagen verstanden, das mindestens eine Modifikation im Kohlenhydratanteil aufweist, über den die kovalente Bindung zur festen Phase erfolgt. Das modifizierte Kollagen kann direkt oder über einen Spacer oder ein Verbindungsglied zur festen Phase kovalent verbunden sein. Beispielsweise kann das Kollagen über Coenzyme, Antikörper oder andere chemische Bindungspartner, welche die kovalente Bindung zur festen Phase formen, an die feste Phase gebunden sein. Unter kovalenter Bindung wird erfindungsgemäß auch eine "quasi-kovalente" Bindung verstanden, wie sie etwa über die sekundären NH₂-Gruppen einer "Covalink™"-Platte (mit Spacer zu Polystyrolplatten) entsteht.

Ein besonders bevorzugtes Adhäsionsprotein, welches mit dem erfindungsgemäßen Verfahren bestimmt werden kann, ist vWF oder eine vWF-Fraktion, insbesondere hochmolekularer vWF, wobei vorzugsweise dessen primäre hämostatische Aktivität (z.B. in einem dynamischen System) ermittelt wird. Andere Beispiele für Adhäsionsproteine umfassen Proteine, welche in vivo mit Kollagen wechselwirken, oder therapeutische Proteine mit einer Affinität zu Kollagen, wie z.B. Fibronektin, Rezeptorproteine von Blutplättchen, Multimeren, Vitronectin, Fibrinogen, Antikörper, oder deren Analoge bzw. Derivate. Prinzipiell können aber sämtliche Proteine oder Substanzen mit einer Affinität zu Kollagen mit dem erfindungsgemäßen Verfahren quantitativ bestimmt werden. Die Bestimmung ist nicht nur eine quantitative oder semiquantitative, sondern auch eine qualitative, da die spezifische Bindungsaktivität als ein Parameter zur Charakterisierung des Adhäsionsproteins herangezogen wird.

Auch ein vWF-Derivat kann mit dem erfindungsgemäßen Verfahren bestimmt werden.

Bevorzugterweise wird als avides Kollagen ein natives Kollagen oder Kollagenderivat eingesetzt, welches eine Bindungskapazität von mindestens einer RCoF-Einheit vWF/mg, vorzugsweise mehr als 10 RCoF-Einheiten/mg, am meisten bevorzugt mehr als 100 RCoF-Einheiten/mg, aufweist.

Erfindungsgemäß wird auch bevorzugterweise ein natives Kollagen oder Kollagenderivat mit einer Bindungskapazität von mindestens 13 µg vWF-Antigen/mg, vorzugsweise mehr als 130 µg/mg, und insbesondere mehr als 1300 µg/mg, an eine feste Phase kovalent fixiert.

Dabei können die Bedingungen von jedem Fachmann so gewählt werden, daß eine Bindung der Substanz an Kollagen ermöglicht wird. Zu den Parametern, unter denen die Bindungsreaktion durchgeführt wird, gehören die Temperatur, die Art des Puffers, der pH-Wert und die Inkubationsdauer. Diese Parameter können zur Durchführung des erfindungsgemäßen Verfahrens von jedem Fachmann ohne Schwierigkeiten ermittelt und eingesetzt werden. Die Inkubation erfolgt dabei vorzugsweise bei einer Temperatur zwischen 15°C bis 37°C, insbesondere jedoch bei Raumtemperatur. Die Auswahl des bei der Durchführung des Verfahrens verwendeten Puffersystems ist dabei nicht eingeschränkt, und es kann ein Puffer mit einem beliebigen pH-Wert oder Ionenstärke verwendet werden. Dies ist insbesondere dann von Vorteil, falls in einem Paralleltest etwa der Antigengehalt des Proteins bestimmt wird und für beide Systeme der gleiche Puffer eingesetzt werden kann.

Zu den erfindungsgemäß verwendeten Kollagenderivaten zählen Fragmente, chemisch modifizierte, strukturell veränderte Proteine bzw. Polypeptide, die von Kollagen abgeleitet sind und Bindungsstellen für die Adhäsionsproteine enthalten, beispielsweise ein Minikollagen (Analytical Biochemistry 231, 57-64 (1995). Das avide Kollagen, welches erfindungsgemäß eingesetzt wird, ist vorzugsweise aus niedermolekularem Kollagen, aus proteolytisch prozessiertem, desintegriertem und homogenisiertem Kollagen ausgewählt. Kollagen humanen Ursprungs, vorzugsweise vom Typ III (insbesondere aus humaner Placenta), stellt dabei ein ganz besonders bevorzugtes Kollagen dar. Jedoch sind auch Kollagene vom Typ I, beispielsweise aus Rindern, Pferden oder Schweinen, zur Verwendung im erfindungsgemäßen Testsystem geeignet.

Gemäß einer besonderen Ausführungsform erfolgt die Bestimmung der Kollagen-bindenden Substanz durch eine spezifische Detektionsreaktion, vorzugsweise durch einen Immunassay. Dabei wird die spezifische Bindung zwischen der Kollagen-bindenden Substanz und dem Kollagen dadurch nachgewiesen, daß die an das Kollagen gebundene Substanz mit einem spezifisch gegen die Substanz gerichteten Antikörper inkubiert wird. Dazu können besonders markierte Antikörper eingesetzt werden, wobei monoklonale Antikörper besonders bevorzugt sind.

Der Komplex aus Kollagen, Kollagen-bindender Substanz und Antikörper kann dann über den Nachweis des gebundenen Antikörpers bestimmt werden. Dies kann durch allgemeine, im Stand der Technik gängige Methoden, wie ein Enzym-, Chromo-, Lumino-, Fluorooder Radioimmunassay, durchgeführt werden. Dazu wird die Bindungsreaktion sowie die Effizienz der Bindung durch die jeweilige Technik ermittelt und die Menge des gebundenen Proteins/der gebundenen Substanz über die Intensität der Färbung, der radioaktiven Strahlung etc. bestimmt. Durch dieses Verfahren kann mit einfachen Mitteln die Funktionalität sowie die Konzentration einer Kollagen-affinen Substanz in einem biologischen Material oder einer Probe festgestellt werden.

Die Bestimmung der gebundenen Kollagen-bindenden Substanz, insbesondere des gebundenen vWF, kann auf vielfältige Weise realisiert werden: beispielsweise durch eine Antigen-Antikörperreaktion unter Verwendung eines markierten Antikörpers, durch die Messung der Änderung der optischen Dichte, durch Aggregometrie oder durch Anwendung einer Lichtstrahlmethode, insbesondere durch dynamische Lichtstreuung, mit welcher der hydrodynamische Radius von vWF/Kollagenpartikel-Komplexen bestimmt werden kann, wobei die Menge an gebundenem vWF dabei direkt mit der ermittelten Größe der Komplexe korrelliert. Daneben können aber auch für die jeweilige Kollagen-bindende Substanz spezifische Verfahren zur Bestimmung der gebundenen Substanz herangezogen werden, z.B. im Falle von vWF oder anderen Blutplättchen-bindenden Proteinen, durch die Bindung von Blutplättchen oder deren Äquivalenten.

Beispielsweise kann mit vWF eine Agglutination von Kollagen-beschichteten Microcarriern, beispielsweise Latex-Kollagen-Partikeln, nach einem aktiven Prinzip induziert werden. Die Agglutination der Partikel ist dabei von der Menge des vWF abhängig. Analog zur Ristocetin-induzierten Agglutination von Thrombozyten durch vWF kann auch hier mit Hilfe eines Aggregometers eine Aggregationskurve erstellt werden, aus der sich die vWF-Konzentration auf Basis der Kollagen-Bindung berechnen läßt.

Gemäß einem besonderen Aspekt der vorliegenden Erfindung wird das erfindungsgemäße Verfahren zur Bestimmung der Funktionalität und Konzentration von Kollagen-bindenden Proteinen, insbesondere von Proteinen mit adhäsiven Eigenschaften oder Aktivitäten eingesetzt. Ein bevorzugtes Protein ist dabei vWF, ein Derivat von vWF, das ein Fragment des kompletten vWF sein kann, das jedoch die spezifischen Kollagen-bindenden Regionen des vWF noch besitzt, eine vWF-Fraktion oder hochmolekularer vWF. Desweiteren kann das erfindungsgemäße Verfahren zur Bestimmung von Fibronektin oder Derivaten davon eingesetzt werden. Es kann jedoch jedes weitere Protein oder ein Fragment oder Derivat davon, das eine Affinität zu Kollagen aufweist, mit dem erfindungsgemäßen Verfahren bestimmt werden. Dazu zählen u.a. Antikörper oder Fragmente davon, Rezeptorproteine, Laminin, Fibrinogen oder Thrombospondin.

Die Quelle, aus der die zu untersuchende Probe mit dem biologischen Material stammt, kann vielfältig sein. Das biologische Material kann natürlichen Ursprungs oder durch ein gentechnisches Verfahren hergestellt sein. Zu biologischen Materialien natürlichen Ursprungs gehören gemäß der Erfindung etwa Blut, Plasma, eine Plasmafraktion, während zu durch gentechnische Verfahren hergestellten Materialien rekombinante Zellkulturen oder Überstände von Zellkulturen gehören.

Das erfindungsgemäße Verfahren eignet sich in besonderer Weise zur Durchführung von Routinetests bei der Bestimmung der Funktionalität von Kollagen-affinen, insbesondere von adhäsiven, hämostatisch aktiven Proteinen und deren Quantifizierung bzw. dem Nachweis der Funktionalität. Eine Anwendung kann dabei erfolgen bei der Bestimmung von vWF bei der Diagnose der vWD und der Differenzierung und Klassifizierung des vWF-Typs, da durch die Sensitivität des erfindungsgemäßen Verfahrens auch nur geringe Abweichungen der vWF-CBA vom Normalzustand festgestellt werden können. Da im Vergleich zu herkömmlichen passiv gekoppelten CBA-Assays, die Bindungskapazität des Kollagens an vWF verbessert ist, ist eine genauere Darstellung des Gehaltes an vWF im Plasma möglich. Der Test kann für alle bisher bekannten Verfahren, bei denen der CBA von vWF bestimmt wird, verwendet werden, wie etwa zum Monitoring von Patienten mit vWD während und nach Behandlung mit Faktor VIII-, vWF-, Faktor VIII/ vWF-Komplex-Präparaten oder Desmopressin, für die Qualitätssicherung von Blutgerinnungsfaktor-Präparaten, insbesondere von Faktor VIII-Komplex- oder vWF-Präparaten, die entweder aus Plasma oder über gentechnologische Verfahren hergestellt sind. Ebenso eignet sich das Testsystem zum Routine-Screening von Kollagenadhäsionsprotein-exprimierenden Klonen in der Biotechnologie, da so effizient eine Auswahl von rekombinanten Klonen, die große Mengen des Proteins exprimieren, getroffen werden kann.

Bisherige Testsysteme mit passiv gekoppeltem Kollagen hatten nur eine begrenzte Kapazität Kollagen-bindender Proteine zu binden, und es wurde schon bei geringen Mengen an z.B. vWF eine Sättigung des immobilisierten Kollagens erreicht. Damit war eine sensitive Differenzierung zwischen rekombinanten Klonen, die unterschiedliche Mengen an Protein exprimieren, nicht möglich. Erst durch die Bereitstellung des erfindungsgemäßen Tests, der eine verbesserte Sensitivität zu Kollagen-bindenden Proteinen aufweist, ist eine einfache Differenzierung von rekombinanten Klonen, die ein Kollagen-bindendes Protein mit unterschiedlicher Stärke exprimieren, möglich.

Eine weitere Anwendung des erfindungsmäßen Verfahrens bietet sich insbesondere bei der Quantifizierung von Kollagen-bindenden Proteinen bei der kommerziellen Herstellung von Blutprodukten aus Plasma, da die Reinheit solcher Produkte innerhalb der Qualitätssicherung gewährleistet sein muß. Durch die verbesserte Sensitivität und Spezifität des erfindungsgemäßen Tests können auch geringste Mengen, etwa an Fibronektin, vWF oder einem anderen Kollagen-bindenden Protein in einem gereinigten Blutprodukt festgestellt werden. Die Sensitivitätsgrenze für den Nachweis eines Kollagen-bindenden Proteins in einer Probe, insbesondere von vWF, liegt dabei vorzugsweise bei 5 ng, besonders bevorzugt bei 1 ng und inbesondere bevorzugt bei 0,5 ng. Die Bindungskapazität beträgt vorzugsweise mindestens 100 ng vWF/µg Kollagen, insbesondere 300 ng/µg Kollagen.

Das erfindungsgemäße Verfahren läßt sich im Vergleich zur Bestimmung des Ristocetin-Cofaktors auch deshalb einfacher standardisieren, da keine Thrombozyten verwendet werden müssen, deren Herkunft das Ergebnis beeinflussen kann. Es stellt auch ein physiologischeres Testsystem dar und bietet weiters eine gegenüber den herkömmlichen Testsystemen stark erhöhte Empfindlichkeit. Die im Stand der Technik üblichen Detektionsgrenzen für CBA werden - wie erwähnt - weit unterboten. So kann eine Detektionsgrenze von unterhalb 30 ng/ml Probe, bevorzugterweise weniger als 20 ng/ml, am meisten bevorzugt weniger als 10 ng/ml, erreicht werden. Darüberhinaus werden die Verhältnisse bei der physiologischen Hämostase simuliert: Die Adhäsion der Thrombozyten an das Kollagen des Endothels/Subendothels sowie deren Aggregation wird von der physiologischen vWF-Aktivität vermittelt.

Das Prinzip der Wechselwirkung des vWF mit an künstlichen Oberflächen fixierten Kollagenen läßt sich auch auf herkömmliche Kollagen-Bindungs-Assays zur Detektion anderer Adhäsionsproteine ausdehnen. Es hat sich überraschenderweise gezeigt, daß die erfindungsgemäßen Systeme, bei denen eine kovalente Bindung zwischen Mikrotiter-Platte und Kollagen geknüpft ist, eine erhöhte Reproduzierbarkeit der Meßergebnisse erlauben.

In einer weiteren Ausführungsform wird Kollagen in der oben dargestellten Weise kovalent an eine Mikrotiterplatte aus Polystyrol oder ein Trägerpartikel gebunden. Nach erfolgter Immobilisierung wird das Kollagen mittels eines proteolytischen Enzyms (z.B. Pepsin und/oder anderer Proteasen mit einem pH-Optimum im sauren Bereich) partiell verdaut, so daß nur jene Strukturen des Kollagens am Träger gebunden bleiben, die dem löslichen Anteil des aviden Kollagens entsprechen. Dadurch wird die Raumstruktur des Kollagens insoweit geändert, daß Bindungsepitope für die später zu quantifizierenden Adhäsionsproteine an der Oberfläche des Trägers exponiert werden.

Der Vorteil dieser Methode besteht darin, daß Kollagen in seiner nativen Struktur an die Trägermatrix gebunden wird und eine vorhergehende Denaturierung, z.B. durch ein proteolytisches Enzym, nicht erforderlich ist. Damit können jene Moleküle, die an natives Kollagen binden, nicht aber mit denaturiertem Kollagen in Wechselwirkung treten können, quantifiziert werden.

In einer weiteren Ausführungsform wird natives Kollagen in einer Suspension an eine aktivierte Trägermatrix kovalent gebunden. Überschüssiges Protein, welches nach dieser Prozedur an der Matrix adsorptiv gebunden vorliegt, wird durch Behandlung mit einem chaotropen oder denaturierenden Reagens (z.B. Harnstoff, Guanidinium-Hydrochlorid, Thiocyanate, Detergentien etc.) durch Waschen der mit Kollagen beschichteten Partikel oder der Näpfe einer Mikrotiterplatte entfernt. Auch hier besteht der Vorteil, daß das Kollagen in einer Struktur fixiert wird, die dem nativen Zustand möglichst ähnlich sein soll.

Für die kovalente Fixierung der aviden Kollagene an geeignete Oberflächen steht ähnlich wie bei der kovalenten Kupplung von Antikörpern an Latex-Partikel eine Vielzahl von Methoden zur Verfügung. So kann beispielsweise eine Fixierung durch direkte Kupplung der primären Aminofunktion der Kollagene an epoxy-aktivierte Partikel erfolgen. Eine weitere Möglichkeit der Immobilisierung besteht in der Carbodiimid-Kupplung an Partikel oder Oberflächen, die eine freie Carboxylgruppe oder primäre Aminogruppe tragen. Die Carbodiimid-Kupplung kann bei pH-Werten von 4 bis 5 durchgeführt werden, ein Bereich, bei denen Kollagene eine besonders hohe Löslichkeit aufweisen. Denkbar ist auch die Verwendung des heterobifunktionellen SPDP-Reagens (Pharmacia, Uppsala, Schweden) sowie allgemein die Kupplung des Kollagens über Spacer-Moleküle.

Weiters lassen sich Maleinsäure-Anhydrid aktivierte Polystyrol-Platten bei neutralem pH-Wert sehr leicht bei Raumtemperatur mit Kollagen unter Ausbildung einer Amidbindung beschichten. Derartige Platten sind beispielsweise unter dem Namen Reacti-Bind™ (Pierce) erhältlich. Zur Beschichtung wird wie erwähnt vorzugsweise Typ-III-Kollagen humanen Ursprungs, insbesondere aus menschlicher Plazenta, verwendet, wobei sich eine Konzentration von nicht mehr als 2 µg/ml als ausreichend erwiesen hat. In einer bevorzugten Ausführungsform des erfindungsgemäßen Assays wird dieser Kollagen-Typ auch als pepsinverdautes Material eingesetzt. Die so beschichteten Platten lassen sich aus wässriger Lösung heraus sehr leicht gefriertrocknen, wonach eine Lagerung der Platten bei 4°C unter Sauerstoff- und Feuchtigkeitsabschluss über mehrere Monate möglich ist. Auf diese Weise hergestellte Mikrotiterplatten eignen sich sehr gut für eine schnelle Durchführung eines Kollagen-Bindungs-Assays für diagnostische Zwecke. Die Nachweisgrenze für vWF liegt dabei in der Größenordnung um 10 bis 20 ng/ml.

Gemäß einem weiteren Aspekt betrifft die vorliegende Erfindung ein Kollagen-Konjugat, bestehend aus einer festen Phase und deren kovalent gebundenem Kollagen, das mindestens eine Modifikation im Kohlenhydratanteil aufweist, über die eine stabile Immobilisierung an einen Träger erfolgt, ohne dass die Bindungskapazität des Kollagens zu einem Kollagen-bindenden Protein im Wesentlichen beeinflusst wird.

Überraschenderweise wurde gefunden, dass es möglich ist, Kollagen chemisch zu modifizieren und modifiziertes Kollagen mittels kovalenter Bindung, an einen Träger zu fixieren, ohne die Affinität zwischen einem Kollagen-bindenden Protein und Kollagen zu beeinflussen, indem eine Modifikation im Kohlenhydratanteil eingeführt wird. Die Bindungskapazität und Bindungseigenschaften des Kollagens zu einem Kollagen-affinen Protein bleiben im erfindungsgemäßen Kollagen-Konjugat insbesondere dadurch unbeeinträchtigt, da gemäß der vorliegenden Erfindung die Modifikation des Kollagens im Kohlenhydratanteil des Moleküls erfolgt, wodurch mögliche Bindungsstellen für adhäsive Proteine, die im Proteinteil des Moleküls liegen, durch Wechselwirkung zwischen Kollagen-Träger über eine direkte Bindung des Proteinteils an den Träger nicht beeinträchtigt werden. Bei bisher im Stand der Technik beschriebenen kovalent gebundenen Kollagen-Konjugaten erfolgte die Bindung über Aminosäurereste des Kollagens, wie etwa bei Wilkinson et al. (1990, Anal. Biochem. 185: 294-296) oder in EP 0 713 707 beschrieben. Dabei konnte nicht ausgeschlossen werden, daß ein Teil der für die Affinität zu Proteinen notwendigen Bereiche des Kollagens blockiert oder die Bindungstellen durch eine Konformationsänderung aufgrund der Kopplung an einen Träger maskiert werden, wodurch die Bindungskapazität des Kollagens zum adhäsiven Protein erniedrigt wird. Das erfindungsgemäße Kollagen-Konjugat umfaßt avides Kollagen und vorzugsweise mindestens eine Modifikation im Kohlenhydratanteil.

Die Modifikation kann dabei durch alle gängigen Verfahren chemischer Art in nicht-modifiziertes, natives Kollagen eingeführt werden, insbesondere durch Oxidation, Sulfatierung, Acylierung, Veresterung, etc. Bevorzugt sind dabei Modifikationen die mindestens eine reaktive Gruppe in den Kohlenhydratanteil des Kollagens einführen, wobei besonders bevorzugt solche Modifikationen sind, die durch eine Oxidation, vorzugsweise der Zuckerreste der Oligosaccharide, wie Galaktose und Glukose, entstehen. Durch die chemische Reaktion oder Modifikation entsteht so ein aktiviertes Kollagen, das in der Lage ist, mit einem weiteren Bindungspartner zu reagieren und eine chemische Verbindung, etwa eine kovalente Bindung einzugehen.

Es wurde festgestellt, daß die spezifische Ausbildung von reaktiven Gruppen im Zuckeranteil des Kollagenmoleküls auch eine kovalente Immobilisierung an einen Träger erlaubt, ohne die Affinität des Kollagens zu Proteinen mit Kollagen-bindenden, adhäsiven Eigenschaften zu beeinträchtigen. Reaktive Gruppen im Kollagen, die kovalente Bindungen eingehen können, können etwa dadurch erhalten werden, daß durch Oxidationsmittel, wie etwa Periodat-(Salze) Zuckerreste, wie Galaktose oder Glukose, im Kohlenhydratanteil des Kollagens zu Aldehydresten oxidiert werden und diese Aldehydgruppe mit anderen, chemisch reaktiven Gruppen eine kovalente Bindung eingehen. Dadurch ist es möglich, chemisch modifiziertes Kollagen an einen Träger stabil kovalent zu binden. Das Kollagen weist daher vorzugsweise im erfindungsgemäßen Kollagen-Konjugat als Modifikation eine reaktive Gruppe, insbesondere eine Aldehydgruppe innerhalb des Kohlenhydratanteils des Kollagens auf. Bevorzugterweise ist das Kollagen gemäß der vorliegenden Erfindung über mindestens eine reaktive Gruppe kovalent an den Träger gebunden.

Als feste Phase können zur Herstellung des erfindungsgemäßen Konjugates alle unlöslichen polymeren Träger eingesetzt werden, die mit den reaktiven Gruppen des modifizierten Kollagens eine kovalente Bindung eingehen können oder nach entsprechender Aktivierung zur kovalenten Bindung fähig sind. Als Trägermaterialien kommen dabei in Betracht organische Polymere, wie Polyamide und Vinylpolymere (Polyacryl, Polystyrol und Polyvinylalkohole und deren Derivate), weiters natürliche Polymere, wie Cellulose, Dextrane, Agarose, Chitin und Polyaminosäure und anorganische Polymere, wie Kieselgel, Glas und Metallhydroxide. Diese Trägermaterialien können in Form von Mikroträgern, Partikeln, z.B. Latex-Partikel, in Form von Membranen, Streifen oder von Platten, wie etwa Mikrotiterplatten, oder für Implantate, vorgefertigte Formen für künstliche Gelenke oder Gelenksverbindungen, oder Wundauflagen, verwendet werden.

In einer besonderen Ausführungsform wird Kollagen kovalent an einen Träger in Form einer Mikrotiterplatte fixiert.

Gemäß einem bevorzugten Aspekt der Erfindung weist die feste Phase im erfindungsgemäßen Kollagen-Konjugat mindestens eine reaktive Gruppe auf, die mit mindestens einer reaktiven Gruppe des modifizierten Kollagens eine kovalente Bindung eingehen kann. Die kovalente Fixierung des modifizierten Kollagens erfolgt dabei direkt über eine Bindung der reaktiven Gruppen.

Funktionell reaktive Gruppen des Trägers sind gemäß der vorliegenden Erfindung solche funktionellen Gruppen, die chemisch aktiviert sind und die mit mindestens einer reaktiven Gruppe des modifizierten Kollagens reagieren können. "Funktionell aktiviert" bedeutet in diesem Zusammenhang chemische Derivatisierung einer oder mehrerer chemischer Gruppen des Trägers, die durch die chemische Reaktion in der Lage sind, mit reaktiven Gruppen des modifizierten Kollagens eine Bindung einzugehen. Funktionell reaktive Gruppen des Trägers können dabei beispielsweise Aldehydgruppen, Anhydridgruppen, Hydrazidgruppen oder Succimidgruppen sein, die mit reaktiven Gruppen des modifizierten Kollagens, wie etwa Aldehydgruppen, eine kovalente Bindung eingehen können. Durch die Verwendung von aktivierten Trägern ist auch gewährleistet, daß über die kovalente Kopplung des modifizierten Kollagens eine möglichst einheitlich beschichtete synthetische Oberfläche zur Verfügung gestellt wird. Zudem kann durch die Bindung zweier reaktiver Gruppen gegebenenfalls eine beschleunigte Herstellung des Konjugates und eine höhere Stabilität erreicht werden.

Gemäß einer besonderen Ausführungsform der vorliegenden Erfindung besteht das erfindungsgemäße Kollagen-Konjugat aus einem modifizierten Kollagen, das als reaktive Gruppe vorzugsweise eine Aldehydgruppe aufweist und einem Träger, der als reaktive Gruppe vorzugsweise eine Hydrazidgruppe aufweist.

Das modifizierte Kollagen wird vorzugsweise in Lösung oder in Suspension an den Träger kovalent gebunden. Durch die stabile, kovalente Bindung des modifizierten Kollagens an den Träger kann dabei nicht-gebundendes und im Überschuß vorhandenes vorliegendes Kollagen leicht entfernt werden, etwa durch einen speziellen Waschschritt.

Gemäß einem weiteren Aspekt der vorliegenden Erfindung kann die feste Phase auch über ein Verbindungsglied oder Spacer mit dem modifizierten Kollagen verbunden sein. Dabei kann Kollagen an ein Antigen, ein Coenzym oder ein Antikörper gekoppelt sein, welches kovalent an der festen Phase gebunden ist. Ist das Verbindungsglied ein Antigen bzw. ein Antikörper, so kann jedes Antigen, zu dem ein spezifischer Antikörper hergestellt werden kann, bzw. jeder Antikörper, der mit einem bestimmten Antigen reagiert, eingesetzt werden. Das Antigen bzw. der Antikörper wird zur Herstellung des erfindungsgemäßen Kollagen-Konjugates derart an Kollagen gebunden, daß durch die chemische Reaktion und der Bindung zwischen modifiziertem Kollagen und Träger, die Bindungseigenschaften des Antigens zum spezifischen Antikörper nicht verloren gehen und die Bindungskapazität von Kollagen zu einem adhäsiven Protein im wesentlichen nicht beeinträchtigt wird. Das Kollagen-Antigen-Konjugat bzw. Kollagen-Antikörper-Konjugat kann gemäß der vorliegenden Erfindung anschließend über eine Antigen-Antikörper-Wechselwirkung an eine feste Matrix gebunden werden. Dazu wird an eine feste Matrix, vorzugsweise eine synthetische Polymermatrix, ein spezifisch gegen das bestimmte Antigen reagierender Antikörper bzw. ein Antigen, gegen das ein Antikörper zur Verfügung steht, gekoppelt, und das Kollagen-Antigen-Konjugat über den matrixgebundenen Antikörper bzw. das Kollagen-Antikörper-Konjugat mit dem matrixgebundenen Antigen an die feste Polymermatrix immobilisiert.

Gemäß einer besonderen Ausführungsform der vorliegenden Erfindung erfolgt die Bindung des modifizierten Kollagens im Kollagen-Konjugat an ein Coenzym. Dieses Coenzym ist insbesondere ein Coenzym, das mit einer bestimmten chemischen Verbindung eine sehr stabile Bindung mit einer Dissoziationskonstante von mindestens K=10⁻¹³ M, vorzugsweise von mindestens K=10⁻¹⁵ M, eingehen kann. Diese Eigenschaft des Coenzyms wird insbesondere dadurch ausgenutzt, daß die chemische Verbindung an eine feste Matrix stabil immobilisiert wird, und über die Matrix-gebundene chemische Verbindung eine stabile Immobilisierung des erfindungsgemäßen Kollagen-Konjugats erfolgt. Das Coenzym im Kollagen-Konjugat ist gemäß einer besonderen Ausführungsform der vorliegenden Erfindung Biotin und die chemische Verbindung, die mit Biotin eine stabile Bindung eingeht, Avidin oder Streptavidin.

Die feste Matrix, an die vorzugsweise Avidin oder Streptavidin gekoppelt ist, wird so über die als Spacer dienende chemische Verbindung mit dem erfindungsgemäßen Kollagen-Konjugat assoziiert. Die feste Matrix kann dabei eine synthetische oder natürliche Polymermatrix sein. Die Polymermatrix kann aus jedem bekannten Trägermaterial, wie schon oben erwähnt, bestehen.

Gemäß einem besonderen Aspekt der Erfindung wird das Kollagen-Biotin-Konjugat an eine feste Matrix in Form einer Mikrotiterplatte, an die Avidin oder Streptavidin gebunden ist über eine Biotin-(Strept)-Avidin-Bindung immobilisiert. Diese Art des erfindungsgemäßen Kollagen-Konjugates ist deshalb bevorzugt, da die Herstellung von Polymermatrices mit daran gekoppeltem (Strept)-Avidin zum allgemeinen Stand der Technik gehört und leicht durchzuführen ist. Durch Einsetzen eines Kollagen-Biotin-Konjugates gemäß der vorliegenden Erfindung kann durch die Bindung, über den Biotin-Streptavidin-Komplex ebenfalls eine einheitlich mit Kollagen beschichtete Matrix zur Verfügung gestellt werden.

Es hat sich überraschenderweise herausgestellt, daß durch das erfindungsgemäße Kollagen-Konjugat, insbesondere durch die chemische Kopplung von im Zuckeranteil modifiziertem Kollagen an eine feste Phase, insbesondere an eine synthetische Oberfläche, sowohl eine wesentlich kürzere Kontaktzeit als auch wesentlich geringere Kollagenmengen verwendet werden müssen, um ein erfindungsgemäßes stabiles Kollagen-Konjugat herzustellen. So wurde gefunden, daß 1 µg des erfindungsgemäßen Kollagens und eine Inkubationszeit von 1 Stunde ausreichen, um Kollagen chemisch an einen Träger, wie etwa eine synthetische Oberfläche zu binden. Durch die wesentlich verkürzte Kontaktzeit aufgrund der chemischen Kopplung von Kollagen an die synthetische Oberfläche kommt es zu keinem Verlust durch Präzipitation an "aktivem" Kollagen und somit reichen wesentlich geringere Kollagenmengen aus, um eine stabile synthetische Kollagenoberfläche zu bilden. Zur Beschichtung des Trägers können daher Kollagenkonzentrationen von weniger als 3 µg/ml, vorzugsweise weniger als 2 µg/ml eingesetzt werden. Die Beschichtung erfolgt für mindestens 10 min bis zu 24 Stunden, bevorzugt ist jedoch eine Beschichtungsdauer von 30 bis 60 min. Die Temperatur kann dabei zwischen 20°C bis 37°C gewählt werden.

Es wurde gefunden, daß es beim erfindungsgemäßen Kollagen-Konjugat zu keiner Aggregierung des Kollagens kommt und daß es auch nach der kovalenten Bindung des Kollagens an einen festen Träger über einen längeren Zeitraum zu keiner Aggregatbildung des immobilisierten Kollagens kommt. Dadurch ist es möglich, ein stabiles Kollagen-Konjugat, das gegebenenfalls an eine feste Polymermatrix gebunden ist, unter Aufrechterhaltung der funktionellen Bindungsstellen für adhäsive Proteine bereitzustellen. Dies ist insbesondere dann von Vorteil, wenn die erfindungsgemäßen Kollagen-Konjugate vor der Weiterverwendung über einen längeren Zeitraum gelagert werden sollen. Die Lagerung kann dabei bei 4°C in Lösung, vorzugsweise jedoch in lyophilisierter Form, über einen Zeitraum von mehreren Monaten oder Jahren erfolgen.

Durch die Stabilität des erfindungsgemäßen Kollagen-Konjugates wird gewährleistet, daß bei Verwendung zu verschiedenen Zeitpunkten nach Herstellung des Kollagen-Konjugates, reproduzierbare Meßdaten ermittelt bzw. direkt miteinander verglichen werden können. Ein Vergleich von Daten, z.B. bei der Bestimmung von vWF:CBA war bisher insbesondere deshalb schwierig, da durch die passive Immobilisierung keine einheitlich beschichteten Kollagen-Oberflächen zur Verfügung standen, und durch die unterschiedliche Verteilung von an die Matrixoberfläche-gekoppeltem Kollagen nur begrenzt eine Aussage zu anderen Meßdaten bzw. zu Verleichsproben oder Kontrollen gemacht werden konnten. Durch die kovalente Kopplung von Kollagen an den Träger kann es auch bei der weiteren Handhabung der synthetischen Kollagenoberfläche, wie z.B. Waschen der Oberfläche mit Lösungen oder Puffern, und im Unterschied zur passiven, unspezifischen Kollagenanlagerung, zu keinem Verlust an Kollagen kommen. Auch besteht in der Verwendung der eingesetzten Lösungen und Puffern eine bessere Flexibilität, da nicht wie bei der passiven Kopplung von Kollagen an eine Oberfläche befürchtet werden muß, daß durch die Änderung des pH-Wertes des Puffers das Kollagen aggreggiert oder ausfällt, wodurch Meßergebnisse oder andere Verfahren, für die das erfindungsgemäße Kollagen eingesetzt wird, stark beeinträchtigt werden. Durch die kovalente Kopplung von Kollagen können darüberhinaus während der Kollagen-Kopplung neutrale bis saure Bedingungen gewählt werden, wodurch die Löslichkeit des Kollagens erhöht wird. Die erhöhte Löslichkeit des Kollagens wiederum wirkt sich positiv auf die einzusetzende Menge an Kollagen aus, die zur Herstellung von Kollagen-beschichteten Trägern notwendig ist. Das erfindungsgemäße Kollagen-Konjugat eignet sich insbesondere zur Durchführung von diagnostischen Tests. Dabei haben sich als Vorteil des Konjugates insbesondere die leichte Standardisierung und die Reproduzierbarkeit von Messergebnissen gezeigt, wodurch ein Vergleich von Ergebnissen ermöglicht wird.

Das erfindungsgemäße Kollagen-Konjugat kann auch zur Herstellung eines Kollagen-beschichteten Implantats, eines künstlichen Gelenks oder einer Gelenksverbindung oder einer Wundauflage, beispielsweise als Kollagen-Vlies, eingesetzt werden, bei denen es von Vorteil ist, dass sie stabil und auf der Oberfläche einheitlich mit Kollagen beschichtet sind. Es sind dabei insbesondere solche Kollagen-Konjugate bevorzugt, die mindestens eine Modifikation im Kohlenhydratanteil aufweisen, über die eine stabile Bindung an die feste Phase erfolgt, ohne die Bindungskapazität zu einer Kollagen-bindenden Substanz zu beeinflussen. Dadurch ist es entweder möglich, zusätzliche, die Wundheilung oder die Verträglichkeit des künstlichen Organs unterstützende Mittel, beispielsweise Fibrinogen oder Fibronektin, an das Kollagen-Konjugat zu binden, oder durch den Erhalt der Bindungsfähigkeit des Kollagen-Konjugates zu während des Wundheilungsprozesses natürlicherweise vorkommenden Kollagen-bindenden Substanzen, den Heilungsprozess durch Verwendung des erfindungsgemäßen Kollagen-Konjugates zu verbessern und/oder zu beschleunigen. Anorganische Polymere als Trägermaterialen können natürlich auch für die Herstellung der erfindungsgemäßen Kollagen-Konjugate eingesetzt werden. Solche Kollagen-Konjugate können beispielsweise als Affinitätsträger bei der Reinigung und Gewinnung von Kollagen-bindenden Substanzen oder zur Entfernung von Kollagen-bindenden Substanzen aus einer Lösung verwendet werden. Dabei bindet die Kollagen-bindende Substanz selektiv an den Affinitätsträger, während nicht-Kollagen-bindende Substanzen in Lösung bleiben; gegebenenfalls wird die Kollagen-bindende Substanz anschließend vom Träger wieder desorbiert. Die Bedingungen zur Durchführung dieses Verfahrens gehören zum allgemeinen Fachwissen und können in analoger Weise, wie etwa in Crouch et al. (1987, Biochem. Biophys. Acta 924:81-86) beschrieben, durchgeführt werden.

Das Kollagen im erfindungsgemäßen Kollagen-Konjugat kann von natürlich vorkommendem oder biotechnologisch oder chemisch hergestelltem Kollagen gewonnen werden, wobei das Kollagen vom Typ I, Typ III, Typ IV, Typ V, Typ VI, Typ VII oder Typ VIII oder deren Derivaten sein kann. Das Kollagen kann humanen, bovinen, porcinen oder equinen Ursprungs sein, wobei jedoch humanes Kollagen, insbesondere des Typs III, bevorzugt ist. Es können jedoch alle von Kollagen abgeleiteten Derivate zur Herstellung des Konjugates eingesetzt werden, sofern sie noch die für Kollagenbindenden Proteine spezifischen Bindungsstellen aufweisen.

Das Kollagen-Konjugat gemäß der vorliegenden Erfindung zeichnet sich insbesondere dadurch aus, daß das im Konjugat an einen Träger kovalent gebundene Kollagen in seinen Bindungseigenschaften sowie seiner Bindungskapazität zu Kollagen-affinen Proteinen, insbesondere zu Proteinen mit hämostatischer Eigenschaft oder Aktivität nicht beeinträchtigt ist. Dies ist insbesondere deshalb wichtig, da das Kollagen-Konjugat bei Verwendung in einem diagnostischen Test durch Beeinträchtigung der Bindungsstellen zu Proteinen eine verringerte Spezifität aufweisen würde. Es wurde jedoch überraschenderweise gefunden, daß im erfindungsgemäßen Kollagen-Konjugat, die Bindungskapazität des Kollagens, insbesondere des modifizierten Kollagens, zu einem affinen Protein nicht beeinträchtigt ist, sondern im Gegensatz dazu die Bindungskapazität des kovalent gebundenen Kollagens im Konjugat zu einem Kollagen-adhäsiven Protein im Vergleich zu Kollagen, das über passive Immobilisierung adsorptiv an einen Träger gebunden ist, wesentlich verbessert und erhöht ist. Dies war deshalb überraschend, da nicht zu erwarten war, daß z.B. im Zuckeranteil des Kollagens modifiziertes Kollagen in gleicher Weise eine affine eine Substanz binden kann wie nicht-modifiziertes Kollagen und daß zudem eine erfindungsgemäße kovalente Immobilisierung von Kollagen an einen Träger die Bindungskapazität des Kollagens nicht negativ beeinflußt. Vielmehr wurde im Rahmen der vorliegenden Erfindung festgestellt, daß durch eine verbesserte und effizientere Kopplungsrate des gebundenen Kollagens an einen Träger, eine synthetische Kollagen-Oberfläche zur Verfügung gestellt werden kann, die weniger Kollagen pro hergestellter Kollagen-Trägeroberfläche benötigt und eine verbesserte Bindungskapazität aufweist.

Eine wesentliche Zielsetzung bei der Durchführung von Substanzuntersuchungen und funktionellen Charakterisierungen besteht in der Bereitstellung einer sehr hohen Empfindlichkeit zum Nachweis von Spuren des Proteins in Substanzgemischen. Das Kollagen-Konjugat in der vorliegenden Erfindung ist insbesondere in der Lage, noch geringste Mengen von beispielsweise vWF in Gemischen nachzuweisen. Dies entspricht einer über 1:2000-fachen Verdünnung von humanem Plasma. Die Sensitivitätsgrenze für den Nachweis eines Kollagen-bindenden Proteins in einer Probe, insbesondere von vWF, liegt daher im erfindungsgemäßen Kollagen-Konjugat bei vorzugsweise 2 ng, besonders bevorzugt 1 ng und insbesondere bevorzugt 0,5 ng vWF.

Das erfindungsgemäß modifizierte Kollagen-Konjugat ist insbesondere dadurch erhältlich, daß nicht-modifiziertes Kollagen einer chemischen Reaktion, unterzogen wird, wodurch es chemisch modifiziert wird. Die chemische Reaktion ist vorzugsweise eine Oxidationsreaktion, bei der insbesondere die Moleküle im Kohlenhydratanteil des Kollagens chemisch verändert werden. Vorzugsweise werden die im Zuckeranteil des Kollagenmoleküls befindlichen Zuckerreste oxidiert, wodurch mindestens eine reaktive Gruppe entsteht. Die Zuckerreste sind vorwiegend Galaktose oder Glukose, bei denen durch den Oxidationsprozeß reaktive Aldehydgruppen entstehen. Mindestens eine reaktive Gruppe des modifizierten Kollagens ist in der Lage, mit mindestens einer reaktiven Gruppe eines Trägers eine kovalente Bindung einzugehen, wodurch ein Kollagen-Konjugat entsteht. Das Kollagen-Konjugat kann gegebenenfalls mit einer festen Matrix assoziiert sein, sofern der Träger im Kollagen-Konjugat nicht selbst eine feste Polymermatrix darstellt.

Gemäß einem weiteren Aspekt betrifft die vorliegende Erfindung auch eine Vorrichtung zur Durchführung des erfindungsgemäßen Bestimmungsverfahrens, welche ein erfindungsgemäßes Kollagen-Konjugat umfaßt. Dieses Kollagen-Konjugat kann einerseits separat von einem Behälter, der zur Aufnahme der Probe geeignet ist, angeboten werden oder selbst als ein Teil des Behälters bzw. als Behälter ausgebildet sein.

Vorzugsweise umfaßt die erfindungsgemäße Vorrichtung auch den Proben-Behälter, also z.B. als Mikrotiterplatte, Küvetten, Perfusionskammern, Kapillaren, deren Ausnehmungen bzw. Oberflächen das kovalent fixierte avide Kollagen enthalten.

Die feste Phase umfassend das immobilisierte avide Kollagen kann aber auch als Körper zur Einführung in einen Proben-Behälter ausgebildet sein, etwa als Kamm passend zu einer Mikrotiterplatte oder als Kügelchen bzw. Granulat. Geeignete Körper sind beispielsweise Mikropartikel, vorzugsweise aus Polystyrol, Latex, Liposomen bzw. Lipidkörper, synthetische Polymere, organische Polymere, wie denaturierte Proteine von Fibrin oder Kollagen, an welchen das avide Kollagen kovalent fixiert ist. Diese Mikropartikel können vorzugsweise markiert sein, beispielsweise durch einen Farbstoff, Fluoreszenzmarker oder durch magnetische Eigenschaften, wobei die Markierung direkt am Partikel angebracht ist oder über einen Vermittler.

Das immobilisierte Kollagen kann zur Bindung des vWF im statischen, aber auch in einem dynamischen Analysensystem eingesetzt werden. Die Bindung des von Willebrand-Faktors an Oberflächen unter Flußbedingungen ist auch von den dabei auftretenden Scherkräften und von der Zusammensetzung der Lösungen, in welchen diese Adhäsion auftritt, abhängig. Entsprechend werden Modelle eingesetzt, bei welchen Suspensionen von Blutplättchen oder Blut durch Kapillaren oder Kammern gepumpt werden, in welchen den perfundierten Lösungen Oberflächen angeboten werden, die mit speziellen Bindungseigenschaften für Proteine aus den Perfusaten ausgestattet sind.

Im Stand der Technik wurden bisher in solchen Perfusionskammern physikalisch mit Kollagen beschichtete Oberflächen verwendet und diese anschließend mit Vollblut perfundiert, wobei die dann resultierenrende Adhäsion von Thrombozyten an die Kollagenschicht durch den von Willebrand-Faktor, der im Vollblut auch enthalten ist, mediiert wird. Die gebundenen Zellen können dann durch konventionelle Färbetechniken nach Fixierung sichtbar gemacht und unter dem Mikroskop qualitativ und auch quantitativ über Mikrodensitometrie beurteilt. Dazu stehen auch Methoden der Bildanalyse über Videosysteme zur Verfügung (Sakariassen et al., Meth. Enzymol. 169, 37-104, 1989). Diese Methoden lassen sich durch das erfindungsgemäße Verfahren insoweit verbessern, als das Kollagen in der Kammer bzw. an der Kapillare kovalent gebunden vorliegt.

Damit wird erstens eine maximale Homogenität der immobilisierten Kollagenschicht erreicht und zweitens verhindert, daß das gebundene Gollagen im Zuge der Perfusion der Kapillaren oder Kammern von der Oberfläche desorbiert.

Gemäß einer bevorzugten Ausführungsform der erfindungsgemäßen Vorrichtung liegt diese in lagerstabiler Form vor, vorzugsweise in gefriergetrockneter Form.

Die vorliegende Erfindung betrifft auch ein Set zur Durchführung des erfindungsgemäßen Verfahrens, welches
a) eine erfindungsgemäße Vorrichtung mit einer festen Phase, an die das avide Kollagen fixiert ist, und
b) eine Komponente enthaltend eine standardisierte Aktivität der Kollagen-bindenden Substanz, vorzugsweise des vWF, enthält.

Als bevorzugter Standard zur Kalibrierung des Testsystems wird ein Referenzplasma oder ein hochgereinigter vWF, insbesondere rvWF, oder eine Fraktion des vWF eingesetzt mit definierter Aktivität.

Die Vorrichtung und die Komponente des erfindungsgemäßen Sets werden vorzugsweise in lagerstabiler Form zur Verfügung gestellt, insbesondere in gefriergetrockneter bzw. lyophilisierter Form.

Weiters kann das erfindungsgemäße Set auch Mittel zur Bestimmung der gebundenen Kollagen-bindenden Substanz, insbesondere des vWF, enthalten. Beispiele für solche Mittel umfassen markierte Antikörperpräparationen, insbesondere monoklonale Antikörper gegen die Kollagen-bindende Substanz, insbesondere gegen ein Adhäsionsprotein, separate Behälter zur Aufnahme von Detektionslösungen und Reagenzien.

Der erfindungsgemäße Testkit kann daher mindestens eine Substanz oder ein Reagens zur Durchführung einer spezifischen Detektionsreaktion, insbesondere zum Nachweis der an Kollagen gebundenen Substanz. Als bevorzugte Substanzen oder Reagentien für die Detektionsreaktion dienen dabei besonders markierte Antikörper, die gegen das Kollagen-affine Protein gerichtet sind, und insbesonderte gegen Kollagen-gebundene Adhäsionsproteine, wobei monoklonale Antikörper besonders bevorzugt sind.

Durch die Bindung des vWF einerseits an Kollagen der Gefäßwand und an Rezeptoren der Blutplättchen andererseits, kommt es zur blutstillenden Wirkung des vWF. Die Bindungen des vWF an den vWF-Rezeptor GP Ib der Blutplättchen erfolgt durch Bindungsstellen innerhalb des Loops Cys 509-Cys 695 des vWF. Überraschenderweise haben die vorliegenden Untersuchungen gezeigt, daß nach der Bindung des vWF an das Kollagen-Konjugat der gebundene vWF mittels einem monoklonalen Antikörper qualitativ und quantitativ nachgewiesen werden kann, der selektiv an die funktionelle Rezeptorbindungsstelle innerhalb des Loop Cys 509-Cys 695 bindet. Damit konnte erstmals ein Testsystem entwickelt werden, das beide funktionell notwendigen Eigenschaften des vWF einbezieht; Bindung an Kollagen und Funktionalität der GP Ib-Rezeptorbindungsstelle.

Besonders bevorzugt enthält der Testkit daher einen monoklonalen Antikörper gegen vWF, vorzugsweise einen monoklonalen Antikörper, der an das funktionelle aktive Epitop des vWF zur Plättchenbindung bindet (Goddall et al. (1985), Brit. J. Haemotol. 59:565-577), und vWF einer bestimmten Aktivität als Standard. Durch die Kombination der Bindung des vWF an eine Kollagenoberfläche einerseits und die Bindung des monoklonalen Antikörpers an das für die vWF-Plättchenbindung verantwortliche Epitop des vWF andererseits, wird in einzigartiger Weise die in vivo-Funktionalität des vWF, Brückenbildung zwischen Kollagen-Epithel und Blutplättchen, simuliert, und somit erstmalig ein neuartiges diagnostisches System dargestellt, welches in vitro die vollständige Funktionalität des vWF hinsichtlich primärer Hämostase berücksichtigt.

Die Detektion erfolgt vorzugsweise nach Reaktion mit einem markierten Substrat, wobei als Marker Fluorogene, Chromogene, radioaktive Substanzen, Luminogene, etc. eingesetzt werden können.

Schließlich betrifft die vorliegende Erfindung auch ein Verfahren zur Herstellung einer erfindungsgemäßen Vorrichtung, welche das Bereitstellen einer Lösung eines Kollagens, das mindestens eine Modifikation im Kohlenhydratanteil aufweist, das chemische Fixieren des Kollagens über die Modifikation im Kohlenhydratanteil an den festen Träger, insbesondere ohne wesentliche Vernetzung des Kollagens, und gegebenenfalls das Gefriertrocknen umfasst. Dabei kann das Verfahren mit sehr niedrigen Konzentrationen des aviden Kollagens durchgeführt werden, vorzugsweise mit weniger als 20 µg avidem Kollagen pro ml Ausgangslösung, insbesondere mit weniger als 10 µg/ml.

Es ist zu betonen, dass das erfindungsgemäße Verfahren gleichermaßen zur quantitativen, semiquantitativen oder qualitativen Bestimmung von plasmatischem bzw. rekombinantem von Willebrand Faktor geeignet ist. So kann als Probe entweder Plasma oder eine Plasmafraktion untersucht werden. Ein wesentlicher Vorteil be-steht jedoch durch die Möglichkeit der Testung von hochkonzentrierten, hochaktiven physiologischen vWF-Präparationen. Gegenstand der vorliegenden Erfindung ist demgemäß ein Verfahren zur Bestimmung der physiologischen Aktivität von vWF in einer Probe, welches dadurch gekennzeichnet ist, dass die Probe mit einem erfindungsgemäßen Kollagen-Konjugat kontaktiert wird und die gebundene Menge an vWF bestimmt wird.

So konnten überraschenderweise spezifische Aktivitäten von min-destens 40 E/mg, bevorzugterweise mehr als 50 E/mg, bis zur theoretischen spezifischen Aktivität getestet werden.

Das erfindungsgemäße Bestimmungsverfahren beinhaltet üblicherweise eine Inkubation des Kollagenimmobilisates mit einer Probe, wobei unterschiedliche Verdünnungen der Probe und gegebenenfalls ein Kalibrator eingesetzt werden. Die Inkubation wird vorzugsweise bei 18 bis 37°C, vorzugsweise bei Raumtemperatur, für einen Zeitraum von 10 min bis 3 h, vorzugsweise 30 min bis 1 h, vorgenommen. Anschließend wird die restliche Probe abgetrennt, gegebenenfalls der feste Träger gewaschen und mit einem Reagens in Kontakt gebracht zur Induktion einer Detektionsreaktion bzw. mit direkten Detektionsmethoden das gebundene Adhäsionsprotein bestimmt. Durch einen Vergleich mit einer Standardpräparation wird die Konzentration des Adhäsionsproteins in der Probe ermittelt.

Schließlich betrifft die vorliegende Erfindung auch die Verwendung des erfindungsgemäßen Verfahrens zur Bestimmung der Funktionalität, insbesondere der Bindungskapazität einer Kollagen-bindenden Substanz, denn das erfindungsgemäße Verfahren eignet sich nicht nur zur Quantifizierung von Proben, sondern aufgrund seiner Spezifität auch z.B. zur Messung von Bindungskapazitäten, wobei insbesondere die ausgezeichnete Standardisierbarkeit des erfindungsgemäßen Verfahrens bedeutende Vorteile gegenüber herkömmlicher Verfahren bietet.

Die Erfindung wird in den nachfolgenden Beispielen und den Zeichnungsfiguren, auf die sie jedoch nicht beschränkt sein soll, näher erläutert.

Es zeigen:
Fig. 1 und 2: Kollagen-Bindungstests unter Verwendung von Mikrotiterplatten, an denen menschliches Typ-III-Kollagen kovalent fixiert ist;
Fig. 3: die Bedeutung des Kollagen-Bindungs-Tests im Hinblick auf strukturelle Änderungen des vWF;
Fig. 4: die Bindung von vWF an kovalent gebundenes modifiziertes Kollagen-Konjugat;
Fig. 5: die Bindung von vWF an Kollagen-Biotin-Konjugat;
Fig. 6: den Vergleich der Bindung von vWF an modifiziertes Kollagen-Konjugat (---) und nicht-modifiziertes Kollagen (.......);
Fig. 7: die Bindung von Fibronektin an kovalent gebundenes modifiziertes Kollagen-Konjugat;
Fig. 8: die Bindung von vWF an modifiziertes Kollagen-Konjugat und Detektion mittels monoklonalem Antikörper mit Spezifität für das funktionell aktive vWF-Epitop;
Fig. 9: die Multimerstruktur von LMW-vWF (Spur b), MMW-vWF (Spur c) und HMW-vWF (Spur d) nach Analyse durch 1% Agarose-Gel-Elektrophorese.
Fig. 10: die Multimerstruktur des vWF aus Citratplasma von Patienten mit vWD, wobei Spur a = Probe #01; Spur b = Probe #02; Spur c = Probe #03; Spur d = Probe #04; Spur e = Probe #05; Spur f = Probe #06; Spur g = Probe #07; Spur h = Probe #08; Spur i = Probe #09; Spur j = Probe #10; Spur k = Probe #11; Spur l = Probe #12; Spur m = Probe #13; Spur n = Normalplasma darstellt; und.

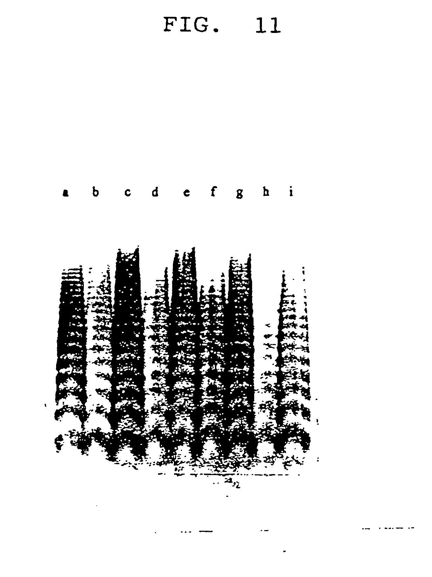

Fig. 11: die Multimerstruktur von vWF vor und nach der Behandlung mit DDAVP, wobei Spur a = Normalplasma, Spur b = Probe #14; Spur c = Probe #14 1 h nach DDAVP-Gabe; Spur d = Probe #15; Spur e = Probe #15 1 h nach DDAVP-Gabe; Spur f = Probe #16; Spur g = Probe #16 1 h nach DDAVP-Gabe; Spur h = Probe #17; Spur i = Probe #17 1 h nach DDAVP-Gabe darstellt.

### Beispiele:

### Beispiel 1:

Zu einer Mikrotiterplatte aus Polystyrol (96 well, Pierce Reactibind™/Maleinsäureanhydrid aktiviert) werden pro Napf 100 µl Suspension von pepsinverdautem Typ III-Kollagen (Southern Biotechnology) aus humaner Plazenta bei einer Konzentration von 2 µg/ml gegeben und 1 h bei Raumtemperatur inkubiert, anschließend für 30 min mit jeweils 150 µl Blockpuffer (Pierce SuperBlock™). Dann werden jeweils 100 µl verschiedener Verdünnungen der von Willebrand-Faktor enthaltenden Probe aufgetragen (25-250 ng vWF/ml). Danach wird mit 100 µl einer Lösung eines Peroxidase-konjugierten anti-vWF-Antikörpers inkubiert (Dakopatts P226, Verdünnung 1:1000). Anschließend werden 100 µl Substrat zugegeben (Single Component TMB Peroxidase EIA Substrate, Bio-Rad) und die Farbreaktion nach 1 min durch 1+1-Verdünnung mit 0,18 M H₂SO₄ abgestoppt. Danach wird die Extinktion bei 450 nm mit einem ELISA-Reader gemessen und die Probenkonzentration gegenüber einer Verdünnungsreihe eines Standards bestimmt. Nach jedem Inkubationsschritt wird jeweils mit 3mal 150 µl-Puffer (Puffer entsprechend der jeweils nächsten Stufe) gewaschen.

### Verwendetes Puffersystem:

- **Puffer 1**: (für die Kollagenbeschichtung und Antikörperverdünnung): 8 mM Natriumphosphat, 135 mM NaCl, 2,6 mM KCl, pH 7,3
- **Puffer 2**: (für Probenverdünnung): entspricht Puffer 1 + 0,05 % Tween 20 und 1 % Rinderserum-Albumin, pH 7,3

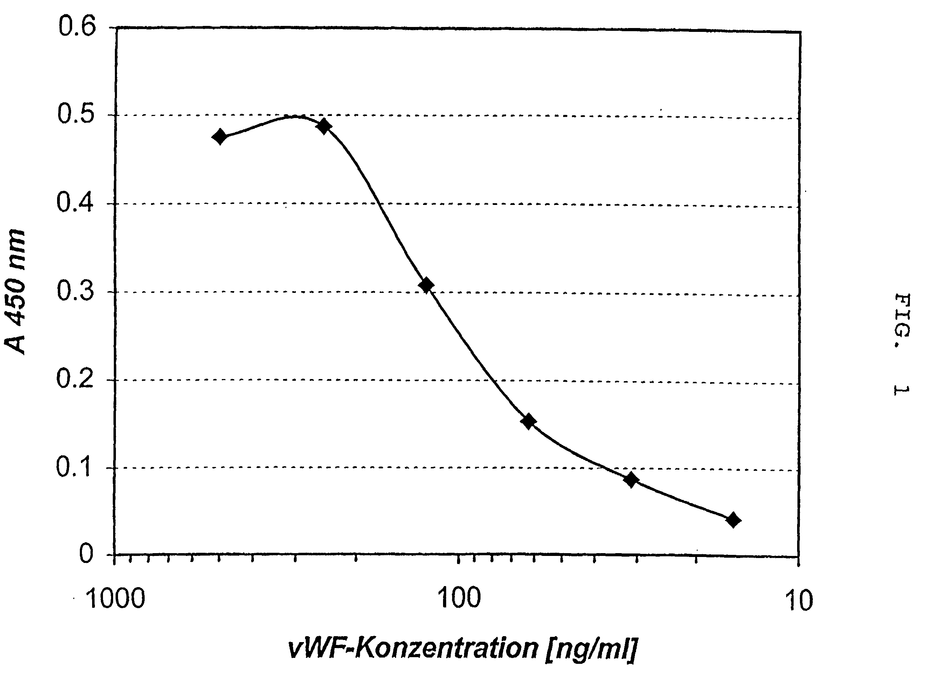

Die Ergebnisse sind der Fig. 1 zu entnehmen. Im Test werden jeweils verschiedene Verdünnungen des vWF eingesetzt und die Absorptionen, wie erwähnt, mittels eines ELISA-Readers bestimmt. Die Figur zeigt die gemessenen Absorptionen als Funktion der im Test eingesetzten vWF-Menge am Beispiel eines Referenzplasmas. Aus einer solchen Kurve, die als Standard dienen kann, und einer Verdünnungsreihe der Probe läßt sich die Kollagen-Bindungs-Aktivität der Probe bestimmen (Parallel Line Assay). Der optimale Arbeitsbereich für diese Variante liegt bei einer Konzentration des vWF im Bereich von 50 bis 250 ng/ml bei einer Nachweisgrenze von 20 ng/ml.

### Beispiel 2:

Ein polyklonaler Antikörper gegen humanen von Willebrand-Faktor (Dakopatts A 082) wird mit Hilfe eines Kits (Amersham RPN 28) biotinyliert. Entsprechend der Vorschrift wird der Antikörper mit 0,05 M Boratpuffer, pH 8,6 auf eine Konzentration von 1 mg/ml verdünnt. Zu 2 ml dieser Lösung werden 80 µl Biotinylierungsreagens gegeben und über eine Stunde bei Raumtemperatur unter leichtem Schütteln inkubiert. Diese Mischung wird anschließend über Sephadex G-25 unter Verwendung von PBS-Puffer, pH 7,5, enthaltend 0,1 % Rinderserum-Albumin, gelfiltriert. Die Fraktionen, die den biotinylierten Antikörper enthalten (Ausschlußvolumen/Messung der Extinktion bei 280 nm), werden gesammelt und unter Zusatz von 0,2 % Natriumazid bei einer Temperatur von 4°C gelagert.

### Beispiel 3:

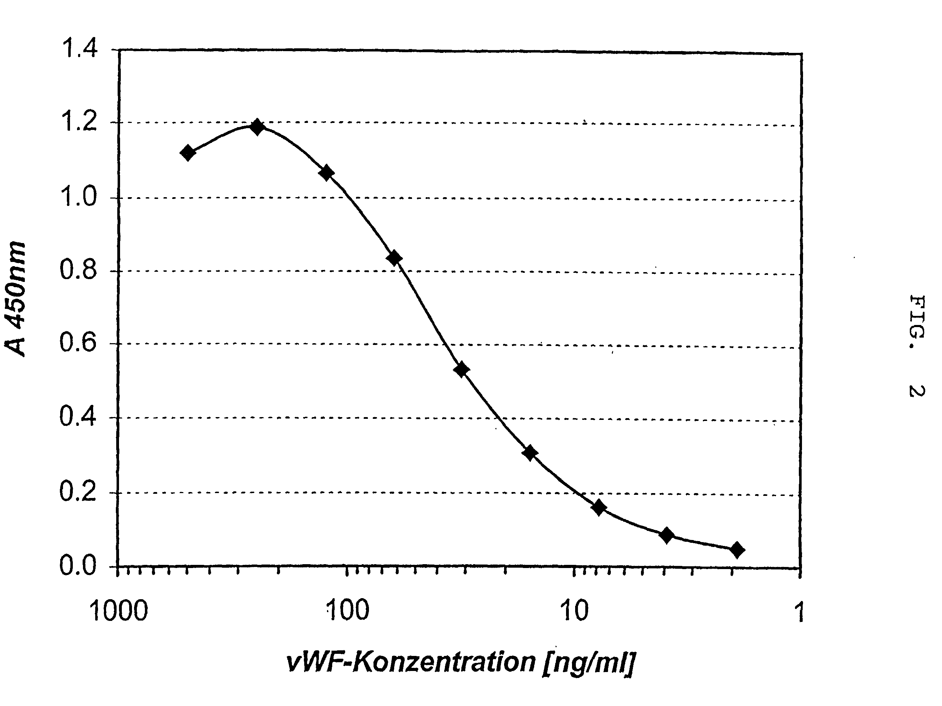

Analog zu Beispiel 1 wird ein vWF-Kollagen-ELISA unter Verwendung eines biotinylierten Antikörpers (hergestellt gemäß Beispiel 2) durchgeführt. Auf eine nach Beispiel 1 präparierte Mikrotiterplatte mit immobilisiertem Kollagen Typ III werden, wie in diesem Beispiel beschrieben, verschiedene Verdünnungen der von Willebrand-Faktor enthaltenden Probe (10 - 200 ng vWF/ml) aufgetragen. Anschließend wird mit jeweils 100 µl einer Verdünnung (1:500 in Puffer 1) eines nach Beispiel 2 biotinylierten Antikörpers über 1 h bei Raumtemperatur inkubiert, danach über 1 h mit jeweils 100 µl eines Strepavidin-Peroxidase Konjugates (Amersham RPN 1231) in einer Verdünnung von 1:1000 (Puffer 1 mit 0,1 % Tween 20). Die Substratzugabe und die weiteren Schritte erfolgen wie unter Beispiel 1 beschrieben. Die Ergebnisse dieser bevorzugten Variante sind in Fig. 2 dargestellt.
Für diese Variante liegt das Optimum zwischen 20 und 200 µg/ml bei einer Nachweisgrenze von 5 µg/ml.

### Beispiel 4:

Analog zu Beispiel 1 wird Typ III-Kollagen aus humaner Plazenta durch Inkubation von 100 µl Kollagen-Suspension bei einer Inkubationszeit von 3 h an eine Mikrotiterplatte kovalent fixiert und Kollagen-Bindungs-Tests werden gemäß den Beispielen 1 und 3 durchgeführt.

### Beispiel 5:

Wie in den Beispielen 1 und 4 beschrieben, wird Typ III-Kollagen aus humaner Plazenta an Mikrotiterplatten kovalent fixiert. Als Beschichtungspuffer dient Puffer 1 unter Zusatz von 0,3% Rinderserum-Albumin. Nach der Beschichtung wird jeweils ein Kollagen-Bindungstest wie unter den Beispielen 1 und 3 beschrieben durchgeführt.

### Beispiel 6:

Wie in den Beispielen 1 und 3 beschrieben, wird ein Kollagen-Bindungs-Test durchgeführt. Die Blockierung der Mikrotiterplatte erfolgt durch Inkubation über 1 h mit 150 µl Phosphatpuffer, pH 7,2 (0,1 M Phosphat, 0,15 M NaCl) unter Zusatz von 3% Rinderserum-Albumin.

### Beispiel 7:

Nach der Beschichtung von Mikrotiterplatten gemäß den Beispielen 1, 4 und 5 mit Kollagen wird nach dem Blockierungsschritt 5mal mit 150 µl 0,1 M Essigsäure gewaschen. Nach Zugabe von weiteren 150 µl 0,1 M Essigsäure pro Well werden die Platten bei -80°C eingefroren und gefriergetrocknet.

### Beispiel 8:

Nach der Beschichtung von Mikrotiterplatten gemäß den Beispielen 1, 4 und 5 mit Kollagen wird nach dem Blockierungsschritt 5mal mit 150 µl H₂O gewaschen. Nach Zugabe von weiteren 150 µl H₂O pro Well werden die Platten bei -80°C eingefroren und gefriergetrocknet.

### Beispiel 9:

Mit Hilfe gemäß den Beispielen 7 und 8 präparierten Mikrotiterplatten wird ein Kollagen-Bindungsassay durchgeführt. Der erste Schritt des Testes besteht in der Inkubation mit der von Willebrand-Faktor enthaltenden Probe wie unter den Beispielen 1 und 3 beschrieben. Alle weiteren Schritte werden ebenfalls wie unter diesen Beispielen beschrieben durchgeführt.

### Beispiel 10:

Zur Bestimmung der Kollagen-Bindungsaktivität eines rekombinanten vWF wird eine Lösung auf eine Einheit RCoF/ml vorverdünnt. Von dieser Lösung sowie von einem Referenzplasma (Fa. Immuno, Wien), das als Standard dient, wird eine 1:50 Verdünnung in Puffer 2 (siehe Beispiel 1) hergestellt sowie davon jeweils weitere 6 Verdünnungen in 1:1,5 Schritten. Diese Verdünnungen werden für die in Beispiel 3 beschriebene Testvariante eingesetzt. Die mit Hilfe eines ELISA-Readers ermittelten Absorptionen werden halblogarithmisch ausgewertet (Koordinatensystem: Absorption linear/Verdünnung logarithmisch). Aus der Verdünnungsreihe des Referenzplasmas (= 100 % vWF:CB) wird eine Bezugskurve erstellt. Aus dieser Bezugskurve lassen sich die Werte für die entsprechenden Probenverdünnungen ablesen. Zur Berechnung der Kollagen-Bindungs-Aktivität (Einheit: vWF:CB) der Probe werden die Werte mit den entsprechenden Probenverdünnungen multipliziert und gemittelt (Parallel Line Assay).

### Beispiel 11:

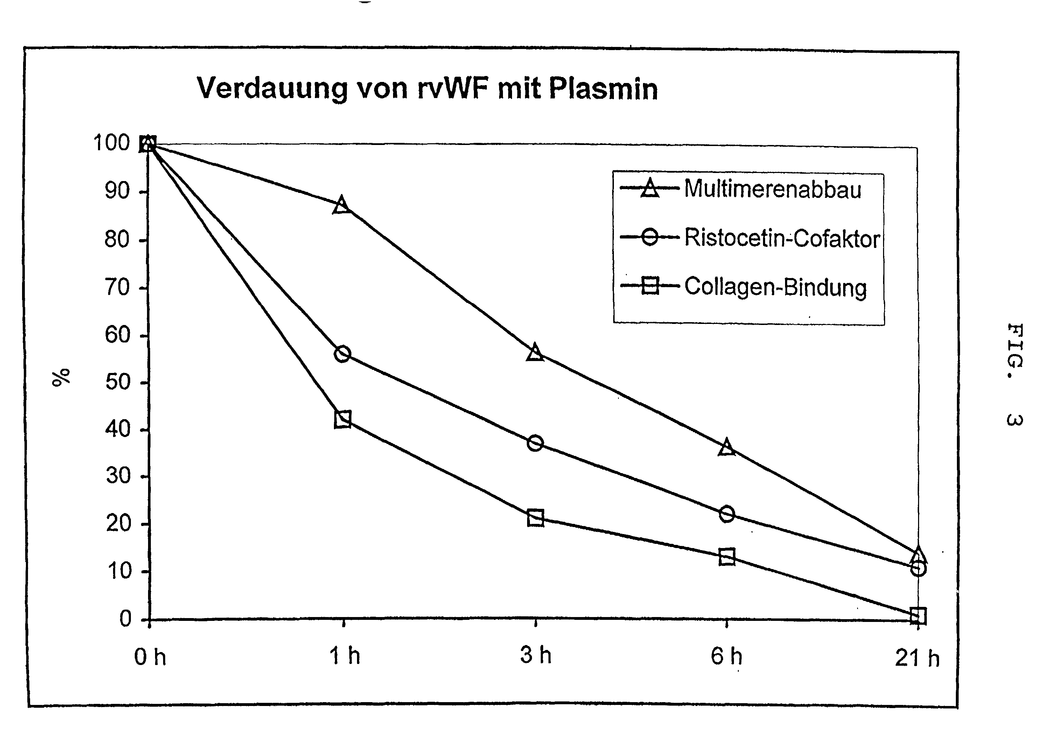

Zu 8,1 ml einer Lösung von rekombinanten vWF (3 E vWF:RCoF/ml) in physiologischem Citrat-Puffer, pH 7,0, werden 0,9 ml einer Lösung von humanem Plasmin (Fa. Chromogenix) mit einer Konzentration von 30 E/ml (Endkonzentration 3 E/ml) zugegeben und bei 37°C inkubiert. Zu den Zeiten 0, 1, 3, 6 und 21 Stunden werden Proben gezogen und die Reaktion durch Zugabe von 0,15 ml einer 10 mM PPACK-Lösung (D-Phe-Pro-Arg-chloromethylketon-dihydrochlorid/Fa. Calbiochem) zu 1,35 ml Probe abgebrochen. Anschließend wird der von Willebrand-Faktor in diesen Proben in Hinblick auf die Multimerenstruktur, der Ristocetin-Cofaktor-Aktivität und der Kollagen-Bindung analysiert. Die Ergebnisse sind in Tabelle 1 angeführt (siehe auch Fig. 3).

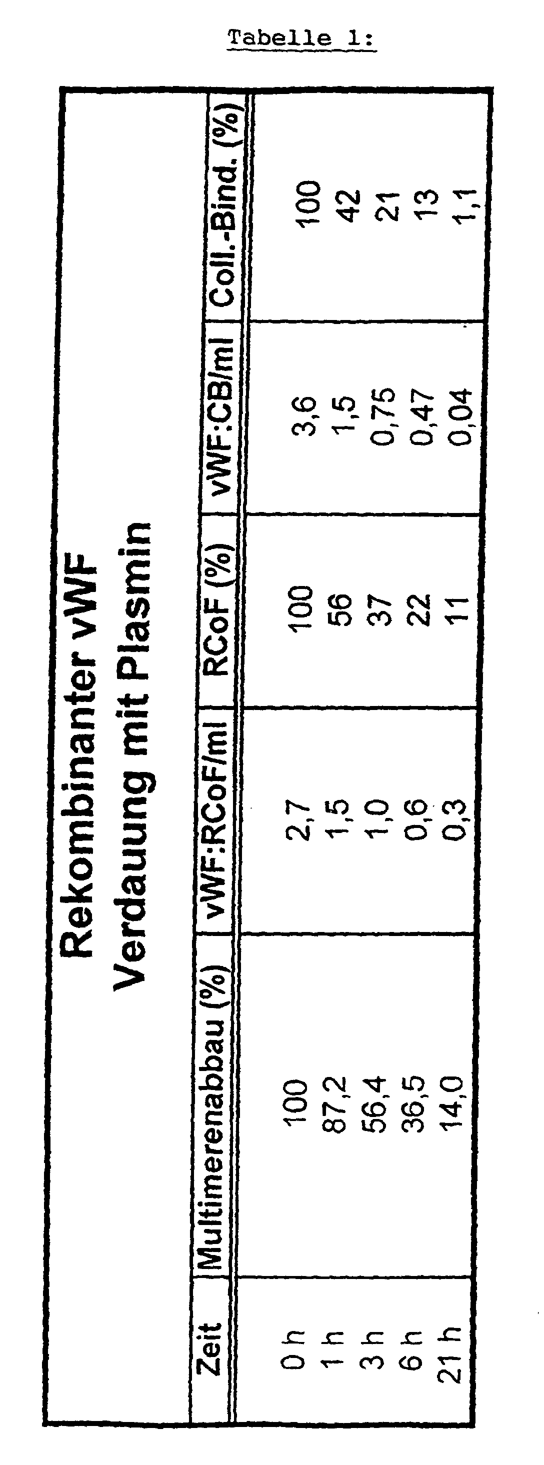

### Beispiel 12:

Die Multimerenstruktur des von Willebrand-Faktors beim enzymatischen Verdau mit Plasmin wird durch SDS-Agarosegelelektrophorese im 2 %igen Agarosegel nach der Methode von Ruggeri et al., Blood 57:1140-1143, analysiert. Dabei werden die von Willebrand-Faktor-Multimere durch eine immunenzymatische Färbung nach Aihara et al., Thromb.Haemostas. 55:263-267, 1986, sichtbar gemacht. Als primärer Antikörper wird ein Kaninchen-anti-von Willebrand-Faktor-Antiserum (Dakopatts, Glostrup, Denmark) in einer Verdünnung von 1:5000 verwendet. Als sekundärer Antikörper dient ein alkalische Phosphatase-konjugierter, affinitätsgereinigter Ziegen-anti-Kaninchen-IgG H + L-Antikörper (Axell, Accurate Chemical and Scientific Corp., NY) in einer Verdünnung von 1:1000. Die Färbung der Proteinbanden erfolgt mittels des Nitroblautetrazoliumchlorid-Bromchlorindolylphosphat-Substrat-Systems. Anschließend werden die gefärbten Banden mit einem Densitometer, Scanner Sharp JX325, gescannt, und die Multimerenbande, welche dem Pentamer des primären Dimers entspricht, mittels der ImageMaster™ Software (Pharmacia) quantifiziert. Die Abnahme der Bande über die Zeit wird in Relation zum Zeitpunkt 0 (= 100 %) bestimmt.

### Beispiel 13:

Die Bestimmung des Ristocetin-Cofaktors erfolgt mit Hilfe einer turbidometrischen Methode unter Verwendung eines 4-Kanal-Aggregometers (Fa. Chronolog) mit angeschlossenem Schreiber. Zur Durchführung der Bestimmung wird von einem Referenzplasma (kalibriert auf Ristocetin-Cofaktor-Aktivität gegenüber dem WHO-Standard) sowie von den vWF enthaltenden Proben durch Verdünnung mit physiologischem Citratpuffer, pH 7,35 (Zusatz von 5 % Humanalbumin) eine Verdünnungsreihe hergestellt. Dann werden in die Küvette des Aggregometers 400 µl formaldehydfixierte Thrombozyten (250.000/µl) mit 50 µl Probe für 3 min bei 37°C und ständigem Rühren (900 U/min) inkubiert. Durch Zugabe von 50 µl einer Lösung von Ristocetin-Sulfat (Apothekernes Laboratorium A.S.) in H₂O (Konzentration 10 mg/ml) wird die Agglutinationsreaktion gestartet, die photometrisch detektiert und vom Schreiber aufgezeichnet wird. Gemessen wird die maximale Reaktionsgeschwindigkeit an der steilsten Stelle der Kurve. Aus der Verdünnungsreihe des Referenzplasmas läßt sich eine Standardkurve erstellen, an der die Ristocetin-Cofaktor-Aktivitäten (Einheit: vWF:RCoF/ml) der Proben abgelesen werden können. Die Ergebnisse sind in Fig. 3 eingetragen. Diese Fig. zeigt die Bedeutung des Kollagen-Bindungstests im Hinblick auf strukturellen Änderungen des vWF im Vergleich zur Bestimmung des Ristocetin-Cofaktors (Beispiel 13) am Beispiel der Kinetik einer Plasmin-Verdauung von rekombinantem vWF (Beispiel 11). Als Vergleich ist in dieser Grafik der prozentuale Abbau der Multimere (Beispiel 12) eingesetzt. Die Fig. sowie die Daten der Tabelle verdeutlichen, daß zwar die Kollagen-Bindungs-Aktivität als auch der Ristocetin-Cofaktor mit steigendem Abbau der Multimeren abnimmt, die Bestimmung der Kollagen-Bindungsaktivät aber ein Testsystem darstellt, das sich von der Ristocetin-Cofaktor-Aktivität deutlich unterscheidet.

### Beispiel 14:

Für die Verwendung in einer Flußkammer wird an das die spezifische Bindungsoberfläche darbietende Deckglas Kollagen vom Typ I mit folgender Methode immobilisiert:

Das Deckglas wird 1 h in 3 %iger HNO₃ bei 90°C behandelt, anschließend mit Wasser solange gewaschen, bis in der Waschlösung keine Säure mehr nachweisbar ist. Anschließend wird das Glas mindestens 36 h in destilliertem Wasser gelagert. Das Deckglas wird dann mit einer 10 %igen wässrigen Lösung von 3-Aminopropyltriethoxysilan versetzt und der pH-Wert der Lösung mit 6 M HCl auf 3,5 eingestellt. Nun wird für 2 h auf 75°C erhitzt, das Deckglas wird aus dem Bad entfernt, mit Wasser vom überschüssigen Reagens durch intensives Waschen befreit und 15 h bei 115°C getrocknet. Durch die Behandlung mit dem Aminosilan werden primäre Aminogruppen an der Glasoberfläche kovalent eingeführt, welche im nächsten Schritt mit Glutardialdehyd weiter derivatisiert werden. Durch Behandlung mit einer 10 %ig wäßrigen Glutardialdehyd-Lösung für 4 h bei 22°C werden mit den primären Aminogruppen Schiff'sche Basen gebildet. Das überschüssige Glutardialdehyd wird durch Waschen mit Eiswasser entfernt. Als Resultat dieser Vorgangsweise liegen aktive Aldehydgruppen an der Oberfläche vor, an welche nun Kollagen kovalent gebunden werden kann. Dazu wird eine Lösung von 1 mg/ml Kollagen, Typ I, aus der Pferdesehne (Kollagenreagens Horm, Nycomed Arzneimittel, München) in 0,1 M Acetatpuffer, pH 4,0, hergestellt. Das derivatisierte Deckglas wird nun 15 h bei 22°C in dieser Lösung inkubiert. Anschließend wird das überschüssige Protein durch Behandeln mit Acetatpuffer entfernt. Das so vorbehandelte Deckglas wird nun als Oberfläche in eine Flußkammer eingesetzt, die entsprechend einer Publikation von Sakariassen et al. (J.Lab.Clin. Med.102, 522-535, (1983)) hergestellt wurde.

### Beispiel 15:

Mit einem nach Beispiel 13 hergestellten Flußkammer-System wird ein Perfusionsexperiment durchgeführt. Als Perfusat dient dazu ein Citrat-Vollblut (Sakariassen et al., Meth.Enzymol. 169, 37-70, (1989)). Die nachfolgende Perfusion sowie die Auswertung der Daten erfolgt gemäß Boomgaard et al. (Vox Sang. 66, 18-24, (1994)). Das Perfusat wird bei einer Durchflußgeschwindigkeit von 95 ml/min und einer Temperatur von 37°C über einen Zeitraum von 5 min durch die Kammer gepumpt. Danach wird das mit Kollagen beschichtete Deckglas der Kammer entnommen, die adhärierten Zellen mit 0,5 % Glutaraldehyd fixiert und mit May-Grünwald-Giemsa-Lösung (Merck, Darmstadt) gefärbt. Die Evaluierung der prozentualen Belegungsdichte der Thrombozyten an der Deckglasoberfläche erfolgt lichtmikroskopisch bei 1000facher Vergrößerung unter Verwendung eines Bildanalysesystems.

### Beispiel 16 : Auflösen von Kollagen

Humanes Kollagen Typ III wurde zu 5 mg/ml in 500 mM Essigsäure bei 4°C aufgelöst und anschließend mit Wasser auf 1 mg/ml verdünnt. Das gelöste Kollagen wurde bei -20°C gelagert.

### Beispiel 17 : Chemische Modifikation von Kollagen

Zur chemischen Modifikation wurde Kollagen (Beispiel 16) in 10 mM Na-Acetatpuffer, pH 4, zu 10 µg/ml verdünnt. Zu 10 ml der Lösung wurden 32 mg NaIO₄ zugegeben und die Modifikation für ½ Stunde bei Raumtemperatur durchgeführt, in der es zu einer chemischen Modifikation und zur Herausbildung von reaktiven Aldehydgruppen im Kollagenmolekül kommt.

### Beispiel 18 : Chemische Kopplung von modifiziertem Kollagen

Zur Herstellung eines an eine synthetische Oberfläche kovalent gebundenen Kollagens wurde modifiziertes Kollagen mit einer Hydrazid-Oberfläche in Kontakt gebracht. Dazu wurde Kollagen nach Beispiel 17 an Mikrotitrationsplatten mit Hydrazid-Oberfläche (Fa. Costar) kovalent gebunden. Je 100 µl der Lösung mit modifiziertem Kollagen wurden je Vertiefung der Mikrotitrationsplatten aufgetragen und für 1 Stunde bei Raumtemperatur inkubiert. Dann wurde 3 x mit 10 mM Tris/HCl-Puffer, pH 7,2, enthaltend 150 mM NaCl und 0,05 % Tween 20, gewaschen. Anschließend wurde die Mikrotitrationsplatte deaktiviert durch Zugabe von 50 mM Tris/HCl-Puffer, pH 8,2, enthaltend 1 % Rinderalbumin. Danach wurde die Deaktivierungslösung entfernt.

### Beispiel 19 : Biotinylierung von Kollagen

Zur Gewinnung von biotinyliertem Kollagen wurde 1 ml gelöstes Kollagen (1 mg/ml in 100 mM Essigsäure) mit 1 ml NaIO4 (20 mM in 100 mM Na-Acetatpuffer, pH 5,5) gemischt und für ½ Stunde inkubiert. Danach wurde 3 µl Glycerin zugegeben und der Überschuß an Glycerin und NaIO₄ durch Gelfiltration abgetrennt. Anschließend wurde Biotin-LC-Hydrazid bis zu einer Endkonzentration von 5 mM zugegeben und für 2 Stunden inkubiert. Der Überschuß an Biotin wurde durch Dialyse entfernt. Durch die Oxidation der Oligosaccharide des Kollagens entstehen reaktive Aldehydgruppen, die mit reaktiven Amingruppen, wie Hydrazid, reagieren und an diese kovalent binden, wodurch ein stabiler Komplex aus Biotin und Kollagen entsteht.

### Beispiel 20 : Kopplung von Biotin-Kollagen an Mikrotiterplatten

Zur Herstellung einer synthetischen Kollagen-Oberfläche wurde kovalent gebundenes Biotin-Kollagen mit einer Streptavidin-haltigen Oberfläche in Kontakt gebracht, damit sich der Biotin-Kollagen-Komplex über die hochaffine Bindung zwischen Biotin und Streptavidin an die synthetische Oberfläche anlagert. Dazu wurde Biotin-Kollagen nach Beispiel 19 an Mikrotitrationsplatten mit Streptavidinoberfläche gebunden. 100 µl Biotin-Kollagen (10 µg/ml in 10 mM Tris/HCl-Puffer, pH 7,4, enthaltend 150 mM NaCl und 0,05% Tween 20), wurden je Vertiefung der Mikrotitrationsplatten aufgetragen und für 1 Stunde bei Raumtemperatur inkubiert. Dann wurde 3 x mit 10 mM Tris/HCl-Puffer, pH 7,2, enthaltend 150 mM NaCl, 0,1% BSA und 0,05 % Tween 20, gewaschen. Anschließend wurde die Mikrotitrationsplatte deaktiviert durch Zugabe von 50 mM Tris/HCl-Puffer, pH 8,2, enthaltend 1 % Rinderalbumin. Danach wurde die Deaktivierungslösung entfernt.

### Beispiel 21 : Bindung des vWF an Kollagen-Konjugat und Detektion von gebundenem vWF

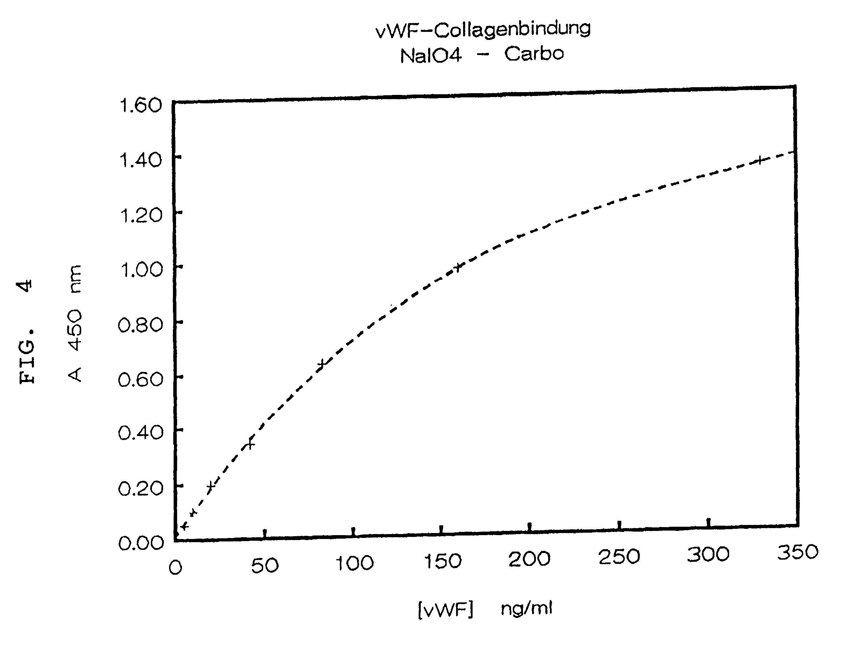

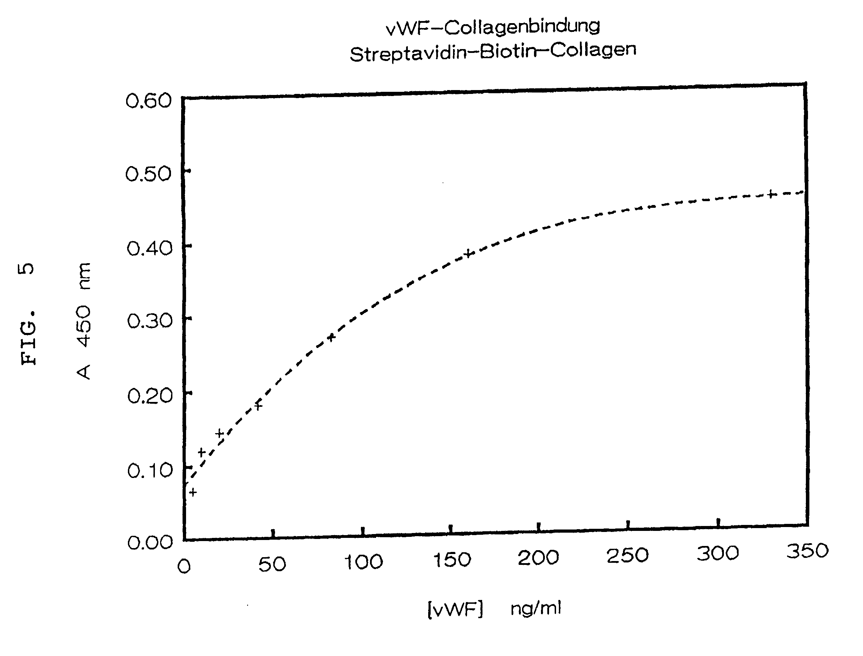

Humanes Citratplasma wurde so verdünnt, daß sich Konzentrationen des vWF zwischen 330 ng/ml und 5 ng/ml ergaben. Als Verdünnungspuffer wurden 10 mM Tris/HCl-Puffer, pH 7,2, enthaltend 150 mM NaCl, 0,05 % Tween 20 und 1 % Rinderalbumin, verwendet. Jeweils 100 µl der Verdünnungen wurden je Vertiefung der Mikrotitrationsplatte mit gekoppeltem modifizierten Kollagen (gemäß Beispiel 18 bzw. Beispiel 19) eingefüllt und für 1 Stunde inkubiert. Dann wurden die Mikrotitrationsplatten 3 x mit 10 mM Tris/HCl- Puffer, pH 7,2, enthaltend 150 mM NaCl und 0,05 % Tween 20, gewaschen. Zur Detektion des an Kollagen gebundenen vWF wurde ein Peroxidase-konjugiertes polyklonales Ziegen-antivWF-Serum (Dako) 1 : 1000 verdünnt und in jede Vertiefung wurden 100 µl aufgetragen. Nach einer Inkubation von 1 Stunde wurde die Mikrotitrationsplatte 3 mal mit 10 mM Tris/HCl-Puffer, pH 7,2, enthaltend 150 mM NaCl und 0,05 % Tween 20, gewaschen. Anschließend wurde durch Zugabe einer Peroxidase-Substratlösung die Farbreaktion gestartet, welche nach 5 Minuten durch Zugabe von 100 µl 1 M H₂SO₄ gestoppt wurde. Die Extinktion der Farblösung wurde dann bei einer Wellenlänge von 450 nm photometrisch bestimmt und in Relation zur verabreichten Konzentration des vWF gesetzt (Figur 4 und Figur 5). Es konnte gezeigt werden, daß die erfindungsgemäße Kollagen-Oberfläche vWF in konzentrationsabhängiger Weise bindet.

### Beispiel 22 : Vergleich der Bindungskapazität von kovalent gebundenem modifizierten Kollagen-Konjugat und passiv immobilisiertem nicht-modifizierten Kollagen

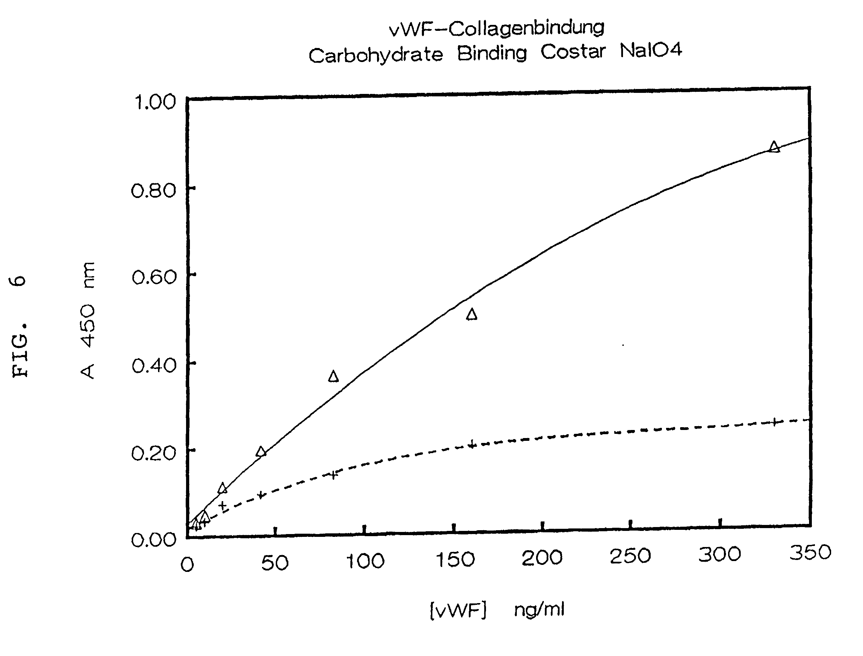

Chemisch modifiziertes Kollagen wurde gemäß Beispiel 18 an Mikrotitrationsplatten mit Hydrazid-Oberfläche (Fa. Costar) kovalent gebunden. Je 100 µl der Lösung mit modifiziertem Kollagen wurden je Vertiefung der Mikrotitrationsplatten aufgetragen und für 1 Stunde bei Raumtemperatur inkubiert. Dann wurde 3 x mit 10 mM Tris/HCl-Puffer, pH 7,2, enthaltend 150 mM NaCl und 0,05 % Tween 20, gewaschen. Anschließend wurde die Mikrotitrationsplatte deaktiviert durch Zugabe von 50 mM Tris/HCl-Puffer, pH 8,2, enthaltend 1 % Rinderalbumin. Danach wurde die Deaktivierungslösung entfernt. In identer Weise wurden die Mikrotitrationsplatten mit passiv immobilisiertem, nicht-modifiziertem Kollagen gemäß dem von Brown et al. (1986, Throm.Res. 43: 303-311) beschriebenen Verfahren behandelt. Anschließend wurde für beide Varianten die vWF-Bindung und vWF-Detektion nach Beispiel 21 durchgeführt. Das Ergebnis ist in Figur 6 dargestellt. Es ist ersichtlich, daß durch die spezifische chemische Modifikation des Kollagens und die nachfolgende kovalente Kopplung in einer wesentlich empfindlicheren vWF-Kollagen-Bindung resultiert als mit nicht-modifiziertem Kollagen, das passiv an die synthetische Oberfläche gebunden ist. Insbesondere fällt auf, daß bei nicht-modifiziertem Kollagen eine Sättigung der vWF-Bindungskapazität bei einer wesentlich geringeren vWF-Konzentration eintritt als beim erfindungsgemäßen Kollagen-Konjugat.

### Beispiel 23 : Charakterisierung von vWF unterschiedlichen Multimerisierungsgrades mittels kovalentem Kollagen-Konjugat und Korrelation zwischen vWF-Multimerisierung und Kollagenbindung

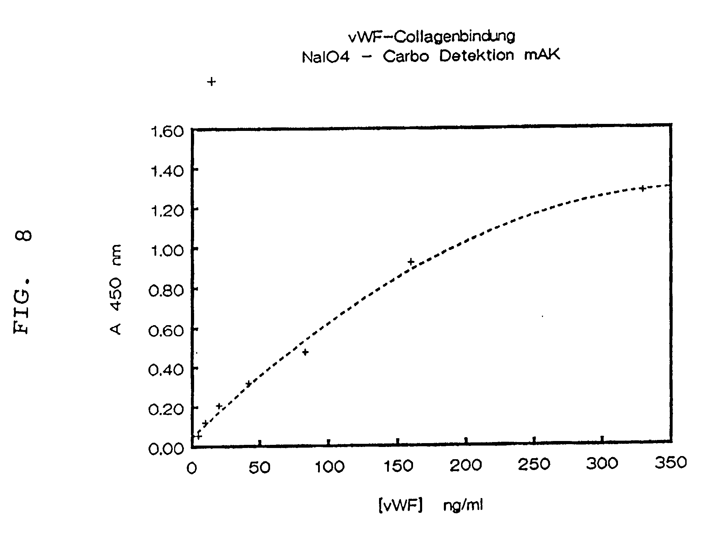

Entsprechend der Deutschen Patentanmeldung DE 44 35 392 wurden ausgehend von humanem Citratplasma Fraktionen des vWF mit unterschiedlichem Multimerisierungsgrad (niedermolekularer vWF (LMW-vWF), mittelmolekularer vWF (MMW-vWF) und hochmolekularer vWF (HMW-vWF)) gewonnen. Fig. 8 zeigt die Multimerisierung von LMW-vWF, MMW-vWF und HMW-vWF. Die vWF-Fraktionen wurden entsprechend Beispiel 21 hinsichtlich der Kollagenbindung untersucht. Als Referenz wurde die Kollagenbindung des vWF aus humanen Plasma verwendet, wobei die Konzentration des vWF-Antigen (vWF:Ag) im humanen Plasma 10 µg/ml beträgt und die Kollagenbindungsaktivität (CBA) des vWF im humanen Plasma in 1 ml als 1 CBA definiert wurde, woraus sich eine spezifische Kollagenbindung von 100 CBA pro 1 mg vWF:Ag ergibt. Gleichzeitig wurde die Funktionalität der vWF-Präparate bezüglich der Ristocetin-Cofaktor-Aktivität (Rist:CoF), sowie der Gehalt an vWF-Antigen (vWF:Ag) ermittelt. Die Ergebnisse sind in Tabelle 2 zusammengefaßt. Es ist ersichtlich, daß eine zunehmende Kollagenbindungsaktivität (CBA/vWF:Ag) mit der Zunahme des Multimerisierungsgrades übereinstimmt. Gleichzeitig ergab sich eine Übereinstimmung zwischen CBA und Rist:CoF.

**Tabelle 2**

| Charakterisierung der Kollagenbindung des vWF in Abhängigkeit des Multimerisierungsgrades | | | | | |
|---|---|---|---|---|---|
| Probe | vWF:Ag (µg/ml) | Rist:Cof (U/ml) | CBA (U/ml) | RistCof/vWF:Ag (U/mg) | CBA/vWF.Ag (U/mg) |
| LMW vWF | 4,1 | 0,04 | 0,046 | 9,7 | 11,2 |
| MMW vWF | 13,7 | 0,4 | 0,652 | 29 | 47,6 |
| HMW vWF | 34,1 | 3,4 | 3,311 | 99 | 97 |

### Beispiel 24 : Bindung von Fibronektin an kovalent gebundenes modifiziertes Kollagen-Konjugat und Detektion des gebundenen Fibronektins

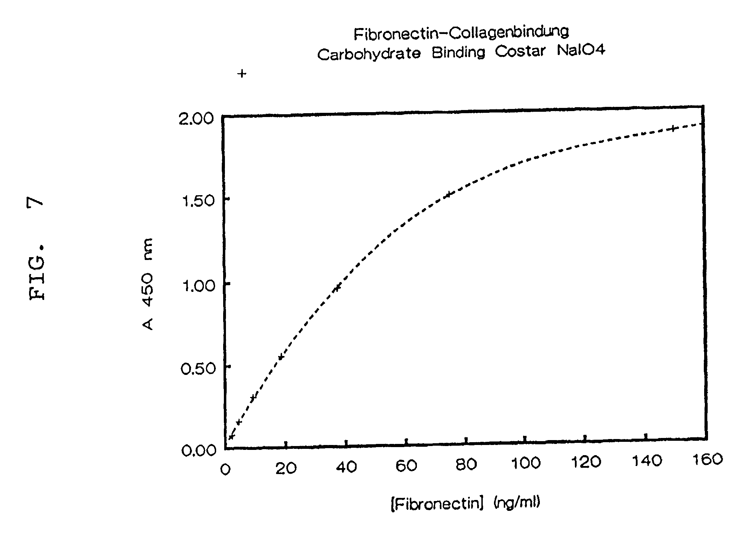

Humanes Citratplasma wurde so verdünnt, daß sich Konzentrationen des Fibronektins zwischen 150 ng/ml und 2,34 ng/ml ergaben. Als Verdünnungspuffer wurde 10 mM Tris/HCl-Puffer, pH 7,2, enthaltend 150 mM NaCl, 0,05 % Tween 20 und 1 % Rinderalbumin, verwendet. Jeweils 100 µl der Verdünnungen wurden je Vertiefung der Mikrotitrationsplatte mit gekoppeltem modifizierten Kollagen (Beispiel 18) eingefüllt und für 1 Stunde inkubiert. Dann wurden die Mikrotitrationsplatten 3 x mit 10 mM Tris/HCl-Puffer, pH 7,2, enthaltend 150 mM NaCl und 0,05 % Tween 20, gewaschen. Zur Detektion des an Kollagen gebundenen vWF wurde ein Peroxidase-konjugiertes polyklonales Ziegen-anti-Fibronektin-Serum (Dako) 1 : 1000 verdünnt und in jede Vertiefung 100 µl dazugegeben. Nach einer Inkubation von 1 Stunde wurde die Mikrotitrationsplatte 3 x mit 10 mM Tris/HCl-Puffer, pH 7,2, enthaltend 150 mM NaCl und 0,05 % Tween 20, gewaschen. Anschließend wurde durch Zugabe einer Peroxidase-Substratlösung die Farbreaktion gestartet, welche nach 5 Minuten durch Zugabe von 100 µl 1 M H₂SO₄ gestoppt wurde. Die Extinktion der Farblösung wurde dann bei einer Wellenlänge von 450 nm photometrisch bestimmt und in Relation zur verabreichten Konzentration des vWF gesetzt (Figur 7). Es wird gezeigt, daß modifiziertes und kovalent gebundenes Kollagen das Plasmaprotein Fibronektin in konzentrationsabhängiger Weise bindet.

### Beispiel 25 : Bindung des vWF an modifiziertes Kollagen-Konjugat und Detektion mittels vWF-Aktivität-spezifischem monoklonalem Antikörper

Humanes Citratplasma wurde so verdünnt, daß sich Konzentrationen des vWF zwischen 330 ng/ml und 5 ng/ml ergaben. Als Verdünnungspuffer wurden 10 mM Tris/HCl-Puffer, pH 7,2, enthaltend 150 mM NaCl, 0,05 % Tween 20 und 1 % Rinderalbumin, verwendet. Jeweils 100 µl der Verdünnungen wurden je Vertiefung der Mikrotitrationsplatte mit gekoppeltem modifizierten Kollagen (Beispiel 18) eingefüllt und für 1 Stunde inkubiert. Dann wurden die Mikrotitrationsplatten 3 mal mit 10 mM Tris/HCl-Puffer, pH 7,2, enthaltend 150 mM Nacl und 0,05 % Tween 20, gewaschen. Zur Detektion des an Kollagen gebundenen vWF wurde ein monoklonaler Antikörper (mAK) verwendet, der selektiv nur an das funktionell aktive Epitop des vWF zur Plättchenbindung bindet (Goodall, A.H. et al., Brit. J. Haematol (1985), 59, 565-577). Nach einer 1-stündigen Inkubation wurden die Mikrotitrationsplatten 3 x mit 10 mM Tris/HCl-Puffer pH 7,2, enthaltend 150 mM NaCl und 0,05 % Tween 20, gewaschen. Zur Detektion des am vWF gebundenen mAK wurde ein Peroxidase-konjugiertes polyklonales Serum aus der Ziege gegen Maus-Immunglobulin (Bio-Rad) 1 : 1000 verdünnt und in jede Vertiefung wurden 100 µl aufgetragen. Nach einer Inkubation von 1 Stunde wurde die Mikrotitrationsplatte 3 x mit 10 mM Tris/HCl-Puffer, pH 7,2, enthaltend 150 mM NaCl und 0,05 % Tween 20, gewaschen. Anschließend wurde durch Zugabe einer Peroxidase-Substratlösung die Farbreaktion gestartet, welche nach 5 Minuten durch Zugabe von 100 µl 1 M H₂SO₄ gestoppt wurde. Die Extinktion der Farblösung wurde dann bei einer Wellenlänge von 450 nm photometrisch bestimmt und in Relation zur verabreichten Konzentration des vWF gesetzt (Figur 8). Der verwendete mAK besitzt eine derartige Bindungsspezifität, daß der mAK nur an das funktionell aktive, für die Plättchenbindung verantwortliche Epitop des vWF bindet. Durch die Kombination der Bindung des vWF an eine Kollagenoberfläche einerseits und die Bindung des mAK an das für die vWF-Plättchenbindung verantwortliche Epitop des vWF andererseits, wird in einzigartiger Weise die in vivo-Funktionalität des vWF, Brückenbildung zwischen Kollagen-Epithel und Blutplättchen, simuliert, und somit erstmalig ein neuartiges diagnostisches System dargestellt, welches in vitro die vollständige Funktionalität des vWF hinsichtlich primärer Hämostase berücksichtigt.

### Beispiel 26: Aufbau eines vWF-Kollagen-Bindungs-ELISA (Enzymimmunologischer in vitro-Test zur quantitativen Bestimung der vWF-Kollagen-Bindung)

### Testprinzip

"Sandwich-Assay": In einer ersten Reaktion bindet vWF der Testprobe an auf dem Teststreifen fixierten Kollagen. In der anschließenden Immunreaktion mit POD-markiertem vWF-Antikörper wird ein Sandwich-Komplex gebildet. Die Menge der Sandwich-Komplexe stellt ein Maß für die Kollagenbindung des vWF des Testmaterials dar. Im nachfolgenden Waschschritt wird das nichtgebundene POD-Konjugat entfernt. Nach Zusatz von H₂O₂ und POD-Substratlösung wird die gebundene POD-Aktivität photometrisch bestimmt.

### Packungsinhalt

1 Mikrotitrationsstreifen, mit kovalent gebundenem Kollagen
2 anti-vWF polyklonales POD-Konjugat (Fa. Dakopatts, Dänemark)
3 POD-Substrat-Tabletten (Fa. Bio-Rad)
4 Puffer-Konzentrat für Probenverdünnung (100 mM Tris/HCl, 1,5 M NaCl, 0,5 % Tween 20, 10 % Albumin)
5 Waschlösungs-Konzentrat (100 mM Tris/HCl, 1,5 M NaCl, 0,5 % Tween 20)
6 vWF-Standard (100 % Normalplasma, IMMUNO AG)

### Zusätzlich erforderlich

- Wasserstoffperoxid (30 %-ig)
- Schwefelsäure (1 N)

### Herstellen der Reagenzien

1. Waschlösungs-Konzentrat 1:10 mit dest. Wasser mischen.
2. Puffer-Konzentrat 1:10 mit dest. Wasser mischen
3. Substratlösung kurz vor Gebrauch ansetzen
4. Antikörper POD-Konjugat 1:1000 mit verdünntem Puffer-Konzentrat verdünnen
5. Verdünnen der Probe:
   1 Teil Citratplasma + 50 Teile verdünntes Puffer-Konzentrat
   1 Teil Citratplasma + 100 Teile verdünntes Puffer-Konzentrat

### Bestimmungsansatz

Wellenlänge: 450 nm
Inkubationstemperatur: +15 -25 °C

In die Vertiefungen der Mikrotitrationsstreifen werden 100 µl verdünnte Probe/Standard pipettiert.

Anschließend wird 1 Stunde inkubiert, dann abgesaugt und 3 x mit 300 µl Waschlösung gewaschen. Nach dem Waschen wird mit 100 µl des verdünnten Antikörpers POD-Konjugat 1 Stunde inkubiert, dann abgesaugt und abermals 3 x mit 300 µl Waschlösung gewaschen. Nach dem erneuten Waschen wird mit 100 µl der POD-Substratlösung exakt 5 Minuten inkubiert, mit 100 µl 1M H₂SO₄ gemischt und die Extinktion innerhalb von 2 Stunden gegen Blankwert gemessen.

### Beispiel 27 :

Eine 96well Mikrotiterplatte (CovaLink™ - Nunc, Roskilde, Denmark) wird mit pepsinverdautem Typ III-Kollagen aus humaner Plazenta (Southern Biotechnology Inc., Birmingham, USA) in einer Konzentration von 3 µg/ml in PBS-Puffer (8 mM Natriumphosphat, 135 mM NaCl, 2,6 mM KCl, pH 7,3) für eine Stunde inkubiert. Danach erfolgt die Blockierung der Platte durch 15 minütiges Inkubieren mit SuperBlock™ Blocking Buffer (Pierce, Rockford, USA).

Anschließend wird die beschichtete Platte mit seriellen Verdünnungen eines Referenzplasmas im Bereich von 1:50 bis 1:1000 in PBS-Puffer (enthaltend 0,05% Tween 20 und 1 % Rinderserum-Albumin) sowie der vWF-enthaltenden Probe für eine Stunde inkubiert. Zum Erstellen der Bezugskurve wird ein Referenzplasma (Immuno AG, Wien, Austria) verwendet, dessen Kollagenbindungsaktivität (vWF:CB) mit einer 1 E/ml definiert ist.

Im nächsten Schritt wird der gebundene vWF durch Inkubation über eine Stunde mit einem polyklonalen Antikörper (biotinylierter Dako A082; Dako, Glostrup, Denmark) in PBS-Puffer detektiert gefolgt von einem Inkubationsschritt mit StreptavidinMeerrettichperoxidase in PBS-Puffer unter Zusatz von 0,1 % Tween 20. Anschließend wird die Substratreaktion unter Verwendung eines chromogenen Substrates (Einkomponenten-Substrat, TMB, Bio-Rad, Hercules, USA) durchgeführt und die Extinktion bei 450 nm mit einem ELISA-Reader gemessen. Alle Schritte dieses Tests erfolgen bei Raumtemperatur mit einem Inkubationsvolumen von 100 µl/well. Nach jedem Inkubationsschritt wird die Platte 3 mal mit 150 µl PBS-Puffer pH 7,3 unter Zusatz von 0,1 % Tween 20 gewaschen.

Die gemäß dieser Vorschrift hergestellte mit Kollagen beschichtete Oberfläche einer Mikrotiterplatte mit kovalent fixierten Spacerarmen wies eine außerordentlich hohe Stabilität auf. Der Test zur Überprüfung einer Bindungsstärke, die der einer kovalenten Bindung entspricht, kann folgendermaßen durchgeführt werden. Nach Behandlung mit 0,4 M NaOH über 15 min bei Raumtemperatur wird die Abnahme der vWF-Bindunaskapazität bestimmt. Die Abnahme betrug weniger als 20% der ursprünglichen Kapazität. Sofern die Abnahme der Bindungskapazität weniger als 30% beträgt, ist die bereitete Oberfläche gemäß der Erfindung geeignet (errechnet aus der Differenz der ELISA-Kurven vor und nach Behandlung mit NaOH).

Das hier verwendete Bestimmungsverfahren läßt sich in Kombination mit der Bestimmung der Multimerenstruktur (Beispiel 12) zur Klassifizierung von vWF-Patienten einsetzen und eignet sich vorzugsweise zur Unterscheidung von Patienten mit den Typen 1 und 2 (Sadler, J.E, Thrombosis and Haemostasis 71(4), 520-525, 1994). Dabei unterscheidet man zwischen einer partiellen, quantitativen Defizienz (Typ 1) bzw. einer qualitativen Defizienz des vWF. Die folgende Tabelle verdeutlicht den Einsatz des Testsystems bei der Klassifizierung der Plasmen von Patienten mit vWF-Disease.

**Tabelle**

| **Patient** | **vWF:RCoFml** **(E/ml)** | **vWF:CB/ml** **(E/ml)** | **Verhältnis** **CB/Ag** | **Anzahl Multimere** | **Typ vWF-Disease** |
|---|---|---|---|---|---|
| **A** | **0,4** | **0,6** | **0,85** | **24** | **1** |
| **B** | **0,5** | **0,6** | **0,98** | **24** | **1** |
| **C** | **< 0,05** | **0,06** | **0,32** | **21** | **2** |
| **D** | **0,07** | **0,07** | **0,35** | **10** | **2** |
| | | | | | |
| **Normal Plasma** | **1,0** | **1,0** | **1,0** | **22 - 25** | |

### Beispiel 28 : Diagnostische Verwendung des Kollagen-Konjugates

Citrat-Plasmen von Patienten mit von Willebrand'scher Krankheit (von Willebrand Disease, vWD) wurden mit einem gemäß einem der Beispiele 16 bis 20 hergestellten Kollagen-Konjugat auf vWF-Kollagenbindung gemäß Beispiel 21 untersucht.

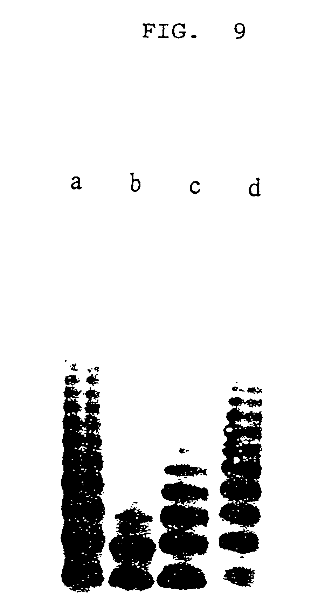

Die Multimerenmuster der untersuchten Plasmen sind in Fig. 9 dargestellt. Die Ergebnisse der CBA-Bestimmung, von Rist:CoF, vWF:Ag, CBA/vWF:Ag und Rist:CoF/vWF:Ag sind in Tabelle 3 zusammengestellt.

**Tabelle 3**

| Funktionelle Charakterisierung der Kollagenbindungs-Aktivität von vWD-Plasmen | | | | | | |
|---|---|---|---|---|---|---|
| Probe | vWD Typ | vWF:Ag (µg/ml) | Rist:Cof (U/ml) | CBA (U/ml) | Rist:Cof/vWF:Ag (U/mg) | CBA/vWF:Ag (U/mg) |
| #01 | 1 | 1,3 | 0,08 | 0,14 | 62 | 107 |
| #02 | 1 | 3,8 | 0,40 | 0,35 | 105 | 92 |
| #03 | 1 | 2,0 | 0,15 | 0,19 | 75 | 95 |
| #04 | 2B | 3,0 | 0,08 | 0,02 | 27 | 7 |
| #05 | 2B | 3,0 | 0,10 | 0,03 | 33 | 10 |
| #06 | 2B | 3,2 | 0,10 | 0,05 | 31 | 16 |
| #07 | 2B | 2,7 | 0,075 | 0,03 | 28 | 11 |
| #08 | 2A | 15,0 | 0,20 | 0,21 | 13 | 14 |
| #09 | 2X | 3,2 | 0,15 | 0,06 | 47 | 19 |
| #10 | 2X | 3,0 | 0,15 | 0,09 | 5 | 30 |
| #11 | 2X | 3,1 | 0,075 | 0,02 | 24 | 6 |
| #12 | 2X | 3,5 | 0,075 | 0,02 | 21 | 6 |
| #13 | 2X | 3,4 | 0,075 | 0,02 | 22 | 6 |

Aus Tabelle 3 und Fig. 9 ist zu erkennen, daß eine klare Korrelation zwischen Multimerisierung, Typ der vWD und der CBA gibt. vWD Typ 1 ist durch verringerte CBA, verringerten vWF:Ag, verringerte Rist:CoF, jedoch normale CBA/vWF:Ag bzw. Rist:CoF/vWF:Ag charakterisiert. Typ 2B vWD ist durch stark verringerte CBA, verringerten vWF:Ag, verringerte Rist:CoF, verringerte CBA/vWF:Ag bzw. Rist:CoF/vWF:Ag charakterisiert und zeigt einen Verlust an hochmolekularen vWF-Polymeren. Typ 2A vWD ist durch verringerte CBA, verringerte Rist:CoF, verringerte CBA/vWF:Ag bzw. Rist.CoF/vWF:Ag charakterisiert und zeigt eine Anreicherung von niedermolekularen vWF-Polymeren. Die Daten der nicht-charakterisierten Patienten (2x) entsprechen der vWD Typ 2B.

### Beispiel 29 : Monitoring von vWD-Patienten während der Verabreichung von DDAVP

Citrat-Plasmen von Patienten mit vWD Typ 1 vor und nach Behandlung mit DDAVP wurden mit einem gemäß einem der Beispiele 16 bis 20 hergestellten Kollagen-Konjugat auf vWF-Kollagenbindung gemäß Beispiel 21 untersucht.

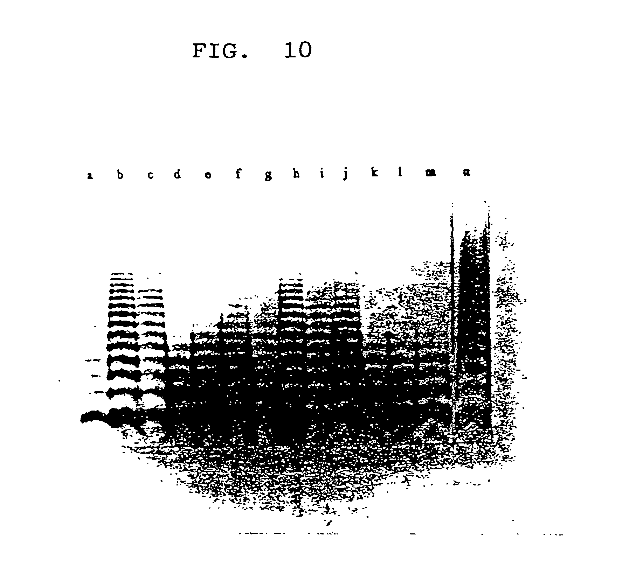

Die Multimerenmuster der untersuchten Plasmen sind in Fig. 10 dargestellt. Die Ergebnisse der CBA-Bestimmung, von Rist:CoF, vWF:Ag, CBA/vWF:Ag und Rist:CoF/vWF:Ag sind in Tabelle 4 zusammengestellt.

**Tabelle 4**

| Funktionnelle Charakterisierung der Kollagenbindungs-Aktivität von Plasmen mit vWD Typ 1 vor und 1 Stunde nach Behandlung mit DDAVP | | | | | |
|---|---|---|---|---|---|
| Probe | vWF:Ag (µg/ml) | Rist:Cof (U/ml) | CBA (U/ml) | Rist:Cof/vWF:Ag (U/mg) | CBA/vVWF:Ag (U/mg) |
| #14 | 7,2 | 0,75 | 0,69 | 104 | 95 |
| # 14 1 h nach DDAVP | 21,9 | 2,125 | 2,82 | 97 | 128 |
| # 15 | 8,6 | 0,6 | 0,61 | 69 | 71 |
| # 15 1 h nach DDAVP | 17,4 | 1,75 | 1,55 | 100 | 89 |
| # 16 r | 9,5 | 0,75 | 0,64 | 78,9 | 67 |
| # 16 1 h nach DDAVP | 22,4 | 1,875 | 1,56 | 83,7 | 69 |
| # 17 | 3,9 | 0,675 | 0,37 | 96 | 95 |
| # 17 1 h nach DDAVP | 11,4 | 1,875 | 1,33 | 164 | 116 |

Fig. 10 und Tabelle 4 zeigen, daß durch die Gabe von DDAVP die Konzentration an vWF:Ag und vWF-Polymeren zunimmt. In korrelierender Weise nimmt die CBA entsprechend zu.

## Patentansprüche

1. Verfahren zur Bestimmung einer Kollagen-bindenden Substanz, insbesondere der Aktivität von einem Adhäsionsprotein in einer Probe, **dadurch gekennzeichnet, dass** Kollagen über eine Modifikation im Kohlenhydratanteil an eine feste Phase kovalent fixiert wird, die Kollagen-bindende Substanz aus der Probe an das Kollagen gebunden wird, und die gebundene Kollagen-bindende Substanz bestimmt wird.

2. Verfahren nach Anspruch 1, **dadurch gekennzeichnet, dass** die primäre hämostatische Aktivität der Probe in einem dynamischen System bestimmt wird.

3. Verfahren nach Anspruch 1 oder 2, **dadurch gekennzeichnet, dass** die primäre hämostatische Aktivität von von Willebrand-Faktor (vWF) bestimmt wird.

4. Verfahren nach einem der Ansprüche 1 bis 3, **dadurch gekennzeichnet, dass** natives Kollagen oder ein Kollagenderivat mit einer Bindungskapazität von mindestens 1 RCoF-Einheiten vWF/mg, vorzugsweise mehr als 10 RCoF-Einheiten/mg, am meisten bevorzugt mehr als 100 RCoF-Einheiten/mg, fixiert wird.

5. Verfahren nach einem der Ansprüche 1 bis 4, **dadurch gekennzeichnet, dass** das native Kollagen oder Kollagenderivat mit einer Bindungskapazität von mindestens 13 µg vWF-Antigen/mg, vorzugsweise mehr als 130 µg/mg, am meisten bevorzugt mehr als 1300 µg/mg, fixiert wird.

6. Verfahren nach einem der Ansprüche 1 bis 5, **dadurch gekennzeichnet, dass** das Kollagen, ausgesucht aus der Gruppe von proteolytisch prozessiertem, desintegriertem und homogenisiertem Kollagen, fixiert wird.

7. Verfahren nach einem der Ansprüche 1 bis 6, **dadurch gekennzeichnet, dass** Kollagen humanen Ursprungs, vorzugsweise vom Typ III, fixiert wird.

8. Verfahren nach einem der Ansprüche 1 bis 7, dadurch gekenn zeichnet, dass die Bestimmung der Kollagen-bindenden Substanz durch eine spezifische Detektionsreaktion, vorzugsweise durch einen Immunassay, erfolgt.

9. Verfahren nach Anspruch 8, **dadurch gekennzeichnet, dass** der Immunassay als Enzym-, Chromo-, Lumino-, Fluoro- oder Radio immunassay durchgeführt wird.

10. Verfahren nach einem der Ansprüche 1 bis 9, **dadurch gekennzeichnet, dass** die gebundene Kollagen-bindende Substanz ein Adhäsionsprotein, insbesondere der gebundene vWF, vWF-Derivat oder Fibronektin ist und durch die weitere Bindung eines markierten Antikörpers bestimmt wird.

11. Verfahren nach einem der Ansprüche 1 bis 10, **dadurch gekennzeichnet, dass** die gebundene Kollagen-bindende Substanz, insbesondere der gebundene vWF, durch die weitere Bindung von Blutplättchen oder deren Äquivalente bestimmt wird.

12. Verfahren nach einem der Ansprüche 1 bis 11, **dadurch gekennzeichnet, dass** die gebundene Kollagen-bindende Substanz, insbesondere der gebundene vWF, durch Messung der Änderung der optischen Dichte bestimmt wird, beispielsweise durch Aggregometrie.

13. Verfahren nach Anspruch 12, **dadurch gekennzeichnet, dass** die gebundene Kollagen-bindende Substanz, insbesondere der gebundene vWF, durch Anwendung einer Lichtstreumethode, insbesondere durch dynamische Lichtstreuung, bestimmt wird.

14. Verfahren nach einem der Ansprüche 1 bis 13, **dadurch gekennzeichnet, dass** als Kollagen-bindende Substanz ein Kollagen-bindendes Protein, insbesondere vWF, ein Derivat davon oder Fibronektin bestimmt wird.

15. Verfahren nach einem der Ansprüche 1 bis 14, **dadurch gekennzeichnet, dass** als Probe biologisches Material natürlichen Ursprungs oder gentechnisch hergestelltes Material eingesetzt wird.

16. Verfahren nach Anspruch 15, **dadurch gekennzeichnet, dass** als biologisches Material Blut, Plasma, eine Plasmafraktion, eine Zellkultur oder ein Zellkulturüberstand verwendet wird.

17. Verfahren nach einem der Ansprüche 1 bis 16, **dadurch gekennzeichnet, dass** die Sensitivitätsgrenze für den Nachweis der Kollagen-bindenden Substanz in einer Probe, insbesondere von vWF, vorzugsweise bei 2 ng, besonders bevorzugt bei 1 ng vWF liegt.

18. Verfahren nach Anspruch 17, **dadurch gekennzeichnet, dass** der die Sensitivitätsgrenze bei 0,5 ng vWF liegt.

19. Kollagen-Konjugat, **dadurch gekennzeichnet, dass** das Kollagen mindestens eine Modifikation im Kohlenhydratanteil aufweist, über die eine stabile Bindung an eine feste Phase erfolgt, wobei die Bindungskapazität des Kollagens zu einer Kollagen-bindenden Substanz nicht wesentlich beeinflusst wird.

20. Kollagen-Konjugat nach Anspruch 19, **dadurch gekennzeichnet, dass** die Modifikation im Kohlenhydratanteil eine Oxidation ist.

21. Kollagen-Konjugat nach Anspruch 19 oder 20, **dadurch gekennzeichnet, dass** das Kollagen als Modifikation eine reaktive Gruppe im Kohlenhydratanteil aufweist.

22. Kollagen-Konjugat nach einem der Ansprüche 18 bis 21, **dadurch gekennzeichnet, dass** die feste Phase mindestens eine reaktive Gruppe aufweist, die mit mindestens einer reaktiven Gruppe Kollagen, insbesondere des modifizierten Kollagens, eine kovalente Bindung eingehen kann.

23. Kollagen-Konjugat nach einem der Ansprüche 19 bis 22, **dadurch gekennzeichnet, dass** die feste Phase ein fester Träger, insbesondere ein natürliches oder synthetisches Polymer, ist.

24. Kollagen-Konjugat nach Anspruch 23, **dadurch gekennzeichnet, dass** das Polymer ein für ein Körper-Implantat, ein künstliches Gelenk oder eine Gelenksverbindung, oder eine Wundauflage, einsetzbares Polymer darstellt.

25. Kollagen-Konjugat nach einem der Ansprüche 19 bis 24, **dadurch gekennzeichnet, dass** die feste Phase eine feste Matrix ist.

26. Kollagen-Konjugat nach einem der Ansprüche 19 bis 25, **dadurch gekennzeichnet, dass** es lagerstabil ist.

27. Kollagen-Konjugat nach einem der Ansprüche 19 bis 26, **dadurch gekennzeichnet, dass** das Kollagen abgeleitet ist vom Typ I, Typ III, Typ IV, Typ V, Typ VI, Typ VII oder Typ VIII.

28. Kollagen-Konjugat nach einem der Ansprüche 19 bis 27, **dadurch gekennzeichnet, dass** das Kollagen vom humanen Kollagen Typ III abgeleitet ist.

29. Kollagen-Konjugat nach einem der Ansprüche 19 bis 28, dadurch erhältlich, dass nicht-modifiziertes Kollagen einer chemischen Reaktion unterzogen wird, wodurch Moleküle des Kohlenhdyratanteils chemisch verändert werden und mindestens eine reaktive Gruppe entsteht, die mit einer, gegebenenfalls aktivierten festen Phase eine kovalente Bindung eingeht.

30. Kollagen-Konjugat nach einem der Ansprüche 19 bis 29, **dadurch gekennzeichnet, dass** das nicht-modifizierte Kollagen einer Oxidationsreaktion unterzogen wird, wodurch Moleküle des Kohlenhydratanteils oxidiert werden.

31. Vorrichtung zur Durchführung eines Verfahrens nach einem der Ansprüche 1 bis 17, **dadurch gekennzeichnet, dass** sie ein Kollagen-Konjugat nach einem der Ansprüche 19 bis 30 umfasst.

32. Vorrichtung nach Anspruch 31, weiters umfassend einen Proben-Behälter zur Aufnahme der Probe, vorzugsweise Näpfchen einer Mikrotiterplatte.

33. Vorrichtung nach Anspruch 31 oder 32, umfassend einen Kamm als feste Phase zur Einführung in den Proben-Behälter.

34. Vorrichtung nach einem der Ansprüche 31 bis 33, umfassend Mikropartikel, vorzugsweise aus Polystyrol oder Latex, als feste Phase.

35. Vorrichtung nach Anspruch 34, **dadurch gekennzeichnet, dass** die Mikropartikel markiert, beispielsweise durch einen Farbstoff, Fluoreszenzmarker oder durch magnetische Eigenschaften sind.

36. Vorrichtung nach einem der Ansprüche 31 bis 35, **dadurch gekennzeichnet, dass** diese in lagerstabiler Form vorliegt, vorzugsweise gefriergetrocknet.

37. Set zur Durchführung des Verfahrens nach einem der Ansprüche 1 bis 17, enthaltend:
a) eine Vorrichtung nach einem der Ansprüche 31 bis 36, und
b) eine Komponente enthaltend eine standardisierte Aktivität der Kollagen-bindenden Substanz, vorzugsweise des vWF.

38. Set nach Anspruch 37, **dadurch gekennzeichnet, dass** die Vorrichtung und die Komponente in lagerstabiler Form vorliegen, vorzugsweise gefriergetrocknet bzw. lyophilisiert.

39. Set nach Anspruch 37 oder 38, **dadurch gekennzeichnet, dass** es weiters ein Mittel zur Bestimmung der gebundenen Kollagen-bindenden Substanz, insbesondere des vWF, enthält.

40. Set nach einem der Ansprüche 37 bis 39, weiters umfassend Substanzen oder Reagenzien für eine spezifische Detektionsreaktion, insbesondere zum Nachweis einer an Kollagen gebundenen Substanz.

41. Set nach Anspruch 40, **dadurch gekennzeichnet, dass** die Substanz zum Nachweis einer Bindung ein Antikörper, vorzugsweise ein monoklonaler Antikörper, ist.

42. Set nach Anspruch 41, **dadurch gekennzeichnet, dass** der Antikörper ein monoklonaler Antikörper ist, der gegen ein funktionell aktives Epitop der Plättchenbindungsstelle des vWF gerichtet ist.

43. Verfahren zur Herstellung einer Vorrichtung nach einem der Ansprüche 31 bis 36, umfassend das Bereitstellen einer Lösung eines Kollagens, das mindestens eine Modifikation im Kohlenhydratanteil aufweist, das chemische Fixieren des Kollagens über die Modifikation im Kohlenhydratanteil an die feste Phase, und gegebenenfalls das Gefriertrocknen.

44. Verfahren nach Anspruch 43, **dadurch gekennzeichnet, dass** die Lösung des aviden Kollagens mit einer Konzentration von weniger als 20, vorzugsweise weniger als 10 µg/ml, bereitgestellt wird.

45. Verfahren zur Bestimmung der physiologischen Aktivität von vWF in einer Probe, **dadurch gekennzeichnet, dass** die Probe mit einem Kollagen-Konjugat nach einem der Ansprüche 19 bis 30 kontaktiert wird, so dass vWF aus der Probe zu dem Kollagen gebunden wird und die gebundene Menge an vWF bestimmt wird.

46. Verwendung des Verfahrens nach einem der Ansprüche 1 bis 17 zur Bestimmung der Funktionalität einer Kollagen-bindenden Substanz.

47. Verwendung eines Kollagen-Konjugates nach einem der Ansprüche 19 bis 30 zur Herstellung eines Implantats, eines künstlichen Gelenks oder einer Wundauflage.

48. Verwendung eines Kollagen-Konjugates nach einem der Ansprüche 19 bis 30 als Affinitätsträger bei der Reinigung und Gewinnung von Kollagen-bindenden Proteinen.

## Claims

1. A method for determining a collagen-binding substance, in particular the activity of an adhesion protein in a sample, **characterised in that** collagen is covalently fixed to a solid phase via a modification in its carbohydrate portion, the collagen-binding substance from the sample is bound to the collagen, and the bound collagen-binding substance is determined.

2. A method according to claim 1, **characterised in that** the primary hemostatic activity of the sample is determined in a dynamic system.

3. A method according to claim 1 or 2, **characterised in that** the primary hemostatic activity of von Willebrand factor (vWF) is determined.

4. A method according to any one of claims 1 to 3, **characterised in that** native collagen or a collagen derivative having a binding capacity of at least 1 RCoF unit vWF/mg, preferably more than 10 RCoF units/mg, most preferred more than 100 RCoF units/mg, is fixed.

5. A method according to any one of claims 1 to 4, **characterised in that** the native collagen or collagen derivative is fixed with a binding capacity of at least 13 µg of vWF antigen/mg, preferably more than 130 µg/mg, most preferred more than 1300 µg/mg.

6. A method according to any one of claims 1 to 5, **characterised in that** collagen selected from the group of proteolytically processed, disintegrated and homogenised collagen is fixed.

7. A method according to any one of claims 1 to 6, **characterised in that** collagen of human origin, preferably of type III, is fixed.

8. A method according to any one of claims 1 to 7, **characterised in that** the determination of the collagen-binding substance is effected by a specific detection reaction, preferably by an immunoassay.

9. A method according to claim 8, **characterised in that** the immunoassay is carried out as an enzyme-, chromo-, lumino-, fluoro- or radio-immunoassay.

10. A method according to any one of claims 1 to 9, **characterised in that** the bound collagen-binding substance is an adhesion protein, in particular the bound vWF, vWF derivative or fibronectin, and that it is determined by the further binding of a labelled antibody.

11. A method according to any one of claims 1 to 10, **characterised in that** the bound collagen-binding substance, in particular the bound vWF, is determined by the further binding of platelets or their equivalents.

12. A method according to any one of claims 1 to 11, **characterised in that** the bound collagen-binding substance, in particular the bound vWF, is determined by measuring the change of the optical density, e.g. by aggregometry.

13. A method according to claim 12, **characterised in that** the bound collagen-binding substance, in particular the bound vWF, is determined by using a light scatter method, in particular by dynamic light scatter.

14. A method according to any one of claims 1 to 13, **characterised in that** a collagen-binding protein, in particular vWF, a derivative thereof, or fibronectin is determined as the collagen-binding substance.

15. A method according to any one of claims 1 to 14, **characterised in that** biological material of natural origin or a material produced by genetic engineering is used as the sample.

16. A method according to claim 15, **characterised in that** blood, plasma, a plasma fraction, a cell culture or a cell culture supernatant is used as said biological material.

17. A method according to any one of claims 1 to 16, **characterised in that** the sensitivity limit for the detection of the collagen-binding substance in a sample, in particular of vWF, preferably is about 2 ng, particularly preferred about 1 ng of vWF.

18. A method according to claim 17, **characterised in that** the sensitivity limit is about 0.5 ng of vWF.

19. A collagen conjugate, **characterised in that** the collagen comprises at least one modification in its carbohydrate portion, via which a stable binding to a solid phase occurs, wherein the binding capacity of the collagen to a collagen-binding substance is not essentially influenced.

20. A collagen conjugate according to claim 19, **characterised in that** the modification in the carbohydrate portion is an oxidation.

21. A collagen conjugate according to claim 19 or 20, **characterised in that** the collagen has a reactive group in its carbohydrate portion as said modification.

22. A collagen conjugate according to any one of claims 19 to 21, **characterised in that** the solid phase comprises at least one reactive group which is capable of forming a covalent bond with at least one reactive group of the collagen, in particular of the modified collagen.

23. A collagen conjugate according to any one of claims 19 to 22, **characterised in that** the solid phase is a solid carrier, in particular a natural or a synthetic polymer.

24. A collagen conjugate according to claim 23, **characterised in that** the polymer constitutes a polymer usable for a body implant, an artificial joint or a joint connection, or a wound cover.

25. A collagen conjugate according to any one of claims 19 to 24, **characterised in that** the solid phase is a solid matrix.

26. A collagen conjugate according to any one of claims 19 to 25, **characterised in that** it is storage-stable.

27. A collagen conjugate according to any one of claims 19 to 26, **characterised in that** the collagen is derived from type I, type III, type IV, type V, type VI, type VII or type VIII.

28. A collagen conjugate according to any one of claims 19 to 27, **characterised in that** the collagen is derived from human collagen type III.

29. A collagen conjugate according to any one of claims 19 to 28, obtainable by subjecting non-modified collagen to a chemical reaction, whereby molecules of its carbohydrate portion are chemically altered and at least one reactive group forms which enters into a covalent bond with an, optionally activated, solid phase.

30. A collagen conjugate according to any one of claims 19 to 29, **characterised in that** the non-modified collagen is subjected to an oxidation reaction, whereby molecules of the carbohydrate portion are oxidised.

31. A device for carrying out a method according to any one of claims 1 to 17, **characterised in that** it comprises a collagen conjugate according to any one of claims 19 to 30.

32. A device according to claim 31, further comprising a sample container for receiving a sample, preferably wells of a microtiter plate.

33. A device according to claim 31 or 32, comprising a comb as a solid phase for introduction into the sample container.

34. A device according to any one of claims 31 to 33, comprising microparticles, preferably of polystyrene or latex, as said solid phase.

35. A device according to claim 34, **characterised in that** the microparticles are labelled, e.g. by a dye, fluorescence marker or by magnetic properties.

36. A device according to any one of claims 31 to 35, **characterised in that** it is provided in storage-stable form, preferably freeze-dried.

37. A kit for carrying out the method according to any one of claims 1 to 17, containing:
a) a device according to any one of claims 31 to 36, and
b) a component containing a standardized activity of the collagen-binding substance, preferably of the vWF.

38. A kit according to claim 37, **characterised in that** the device and the component are provided in storage-stable form, preferably freeze-dried or lyophilized, respectively.

39. A kit according to claim 37 or 38, **characterised in that** it further contains means for determining the bound collagen-binding substance, in particular the vWF.

40. A kit according to any one of claims 37 to 39, further comprising substances or reagents for a specific detection reaction, in particular for detecting a substance bound to collagen.

41. A kit according to claim 40, **characterised in that** the substance for detecting a binding is an antibody, preferably a monoclonal antibody.

42. A kit according to claim 41, **characterised in that** the antibody is a monoclonal antibody which is directed against a functionally active epitope of the platelet binding site of the vWF.

43. A method for producing a device according to any one of claims 31 to 36, comprising providing a solution of a collagen which comprises at least one modification in its carbohydrate portion, chemically fixing the collagen to the solid phase via said modification in its carbohydrate portion, and optionally freeze-drying.

44. A method according to claim 43, **characterised in that** the solution of the avid collagen is provided at a concentration of less than 20, preferably less than 10 µg/ml.

45. A method for determining the physiological activity of vWF in a sample, **characterised in that** the sample is contacted with a collagen conjugate according to any one of claims 19 to 30 so that vWF from said sample is bound to the collagen, and the bound amount of vWF is determined.

46. The use of a method according to any one of claims 1 to 17 for determining the functionality of a collagen-binding substance.

47. The use of a collagen conjugate according to any one of claims 19 to 30 for preparing an implant, an artificial joint or a wound cover.

48. The use of a collagen conjugate according to any one of claims 19 to 30 as an affinity carrier in the purification and recovery of collagen-binding proteins.

## Revendications

1. Procédé de détermination d'une substance liant le collagène, en particulier de l'activité d'une protéine d'adhésion, dans un échantillon, **caractérisé en ce que** le collagène est fixé de manière covalente à une phase solide par une modification de la part en hydrates de carbone, la substance liant le collagène provenant de l'échantillon est liée au collagène et la substance liant le collagène liée est déterminée.

2. Procédé selon la revendication 1, **caractérisé en ce que** l'activité hémostatique primaire de l'échantillon est déterminée dans un système dynamique.

3. Procédé selon la revendication 1 ou 2, **caractérisé en ce que** l'activité hémostatique primaire du facteur de von Willebrand (FvW) est déterminée.

4. Procédé selon l'une des revendications 1 à 3, **caractérisé en ce que** du collagène natif ou un dérivé de collagène ayant une capacité de liaison d'au moins 1 unité RCoF FvW/mg, de préférence supérieure à 10 unités RCoF/mg, de manière préférée entre toutes supérieure à 100 unités RCoF/mg, est fixé.

5. Procédé selon l'une des revendications 1 à 4, **caractérisé en ce que** du collagène natif ou un dérivé du collagène avec une capacité de liaison d'au moins 13 µg d'antigène FvW/mg, de préférence supérieure à 130 µg/mg, de manière préférée entre toutes supérieure à 1 300 µg/mg, est fixé.

6. Procédé selon l'une des revendications 1 à 5, **caractérisé en ce que** du collagène, choisi dans le groupe comprenant le collagène traité par protéolyse, désintégré et homogénéisé, est fixé.

7. Procédé selon l'une des revendications 1 à 6, **caractérisé en ce que** du collagène d'origine humaine, de préférence de type III, est fixé.

8. Procédé selon l'une des revendications 1 à 7, **caractérisé en ce que** la détermination de la substance liant le collagène a lieu par une réaction de détection spécifique, de préférence par immunodosage.

9. Procédé selon la revendication 8, **caractérisé en ce que** le dosage immunologique est réalisé comme dosage immuno-enzymatique, chromo-immunodosage, dosage lumino-immunologique, dosage fluoro-immunologique ou dosage radio-immunologique.

10. Procédé selon l'une des revendications 1 à 9, **caractérisé en ce que** la substance liée liant le collagène est une protéine d'adhésion, en particulier le FvW lié, le dérivé du FvW ou la fibronectine, et qu'elle est déterminée par liaison supplémentaire d'un anticorps marqué.

11. Procédé selon l'une des revendications 1 à 10, **caractérisé en ce que** la substance liée liant le collagène, en particulier le FvW lié, est déterminée par liaison supplémentaire de plaquettes sanguines ou de leur équivalent.

12. Procédé selon l'une des revendications 1 à 11, **caractérisé en ce que** la substance liée liant le collagène, en particulier le FvW lié, est déterminée par mesure de la modification de la densité optique, par exemple par agrégométrie.

13. Procédé selon la revendication 12, **caractérisé en ce que** la substance liée liant le collagène, en particulier le FvW lié, est déterminée par application d'une technique de diffusion de la lumière, en particulier par diffusion dynamique de la lumière.

14. Procédé selon l'une des revendications 1 à 13, **caractérisé en ce que** l'on détermine, comme substance liant le collagène, une protéine de liaison au collagène, en particulier le FvW, un dérivé de ce facteur ou la fibronectine.

15. Procédé selon l'une des revendications 1 à 14, **caractérisé en ce qu'**on utilise comme échantillon un matériau biologique d'origine naturelle ou un matériau fabriqué par génie génétique.

16. Procédé selon la revendication 15, **caractérisé en ce qu'**on utilise comme matériau biologique du sang, du plasma, une fraction plasmatique, une culture cellulaire ou un surnageant de culture cellulaire.

17. Procédé selon l'une des revendications 1 à 16, **caractérisé en ce que** la limite de sensibilité pour la détection de la substance liant le collagène dans un échantillon, en particulier du FvW, est de préférence de 2 ng, de manière particulièrement préférée de 1 ng de FvW.

18. Procédé selon la revendication 17, **caractérisé en ce que** la limite de sensibilité est de 0,5 ng de FvW.

19. Conjugué de collagène, **caractérisé en ce que** le collagène présente au moins une modification de la part d'hydrate de carbone, entraînant une liaison stable à une phase solide, la capacité de liaison du collagène à une substance liant le collagène n'étant pas considérablement influencée.

20. Conjugué de collagène selon la revendication 19, **caractérisé en ce que** la modification de la part d'hydrate de carbone est une oxydation.

21. Conjugué de collagène selon la revendication 19 ou 20, **caractérisé en ce que** le collagène présente comme modification un groupe réactif dans la part d'hydrate de carbone.

22. Conjugué de collagène selon l'une des revendications 19 à 21, **caractérisé en ce que** la phase solide présente au moins un groupe réactif qui peut former une liaison covalente avec au moins un groupe réactif du collagène, en particulier du collagène modifié.

23. Conjugué de collagène selon l'une des revendications 19 à 22, **caractérisé en ce que** la phase solide est un porteur solide, en particulier un polymère naturel ou de synthèse.

24. Conjugué de collagène selon la revendication 23, **caractérisé en ce que** le polymère est un polymère utilisable pour un implant corporel, une prothèse articulaire ou une liaison articulaire, ou un pansement.

25. Conjugué de collagène selon l'une des revendications 19 à 24, **caractérisé en ce que** la phase solide est une matrice solide.

26. Conjugué de collagène selon l'une des revendications 19 à 25, **caractérisé en ce qu'**il est stable à la conservation.

27. Conjugué de collagène selon l'une des revendications 19 à 26, **caractérisé en ce que** le collagène est dérivé des types I, III, IV, V, VI, VII ou VIII.

28. Conjugué de collagène selon l'une des revendications 19 à 27, **caractérisé en ce que** le collagène est dérivé du collagène humain de type III.

29. Conjugué de collagène selon l'une des revendications 19 à 28 que l'on peut obtenir un soumettant le collagène non modifié à une réaction chimique par laquelle les molécules de la part d'hydrate de carbone sont modifiées chimiquement et au moins un groupe réactif est obtenu, qui forme une liaison covalente avec une phase solide le cas échéant activée.

30. Conjugué de collagène selon l'une des revendications 19 à 29, **caractérisé en ce que** le collagène non modifié est soumis à une réaction d'oxydation qui oxyde des molécules de la part d'hydrate de carbone.

31. Dispositif pour la réalisation d'un procédé selon l'une des revendications 1 à 17, **caractérisé en ce qu'**il comprend un conjugué de collagène selon l'une des revendications 19 à 30.

32. Dispositif selon la revendication 31, comprenant en outre un récipient pour échantillon pour recevoir l'échantillon, de préférence les puits d'une plaque de microtitration.

33. Dispositif selon la revendication 31 ou 32, comprenant un peigne comme phase solide pour introduction dans un récipient pour échantillon.

34. Dispositif selon l'une des revendications 31 à 33, comprenant des microparticules, de préférence en polystyrène ou en latex, comme phase solide.

35. Dispositif selon la revendication 34, **caractérisé en ce que** les microparticules sont marquées, par exemple par un colorant, un marqueur de fluorescence ou par des propriétés magnétiques.

36. Dispositif selon l'une des revendications 31 à 35, **caractérisé en ce qu'**il se présente sous forme stable à la conservation, de préférence lyophilisé.

37. Kit pour la réalisation du procédé selon les revendications 1 à 17, contenant :
a) un dispositif selon l'une des revendications 31 à 36, et
b) un composant contenant une activité standardisée de la substance liant le collagène, de préférence le FvW.

38. Kit selon la revendication 37, **caractérisé en ce que** le dispositif et le composant sont sous forme stable à la conservation, de préférence lyophilisée.

39. Kit selon la revendication 37 ou 38, **caractérisé en ce qu'**il contient en outre un moyen pour déterminer la substance liée liant le collagène, en particulier le FvW.

40. Kit selon l'une des revendications 37 à 39, comprenant en outre des substances ou des réactifs pour une réaction de détection spécifique, en particulier pour la détection d'une substance liée au collagène.

41. Kit selon la revendication 40, **caractérisé en ce que** la substance vise à la détection d'une liaison d'un anticorps, de préférence d'un anticorps monoclonal.

42. Kit selon la revendication 41, **caractérisé en ce que** l'anticorps est un anticorps monoclonal, qui est dirigé contre un épitope fonctionnel actif du site de liaison aux plaquettes du FvW.

43. Procédé de fabrication d'un dispositif selon l'une des revendications 31 à 36, comprenant la préparation d'une solution d'un collagène, qui présente au moins une modification de la part d'hydrate de carbone, la fixation chimique du collagène à la phase solide, par modification de la part d'hydrate de carbone, et le cas échéant la lyophilisation.

44. Procédé selon la revendication 43, **caractérisé en ce que** la solution du collagène avide est préparée avec une concentration inférieure à 20, de préférence inférieure à 10 µg/ml.

45. Procédé pour la détermination de l'activité physiologique du FvW dans un échantillon, **caractérisé en ce que** l'échantillon est mis en contact avec un conjugué de collagène selon l'une des revendications 19 à 30, de sorte que le FvW provenant de l'échantillon soit lié au collagène et de sorte que la quantité liée au FvW soit déterminée.

46. Utilisation du procédé selon l'une des revendications 1 à 17 pour déterminer la fonctionnalité d'une substance liant le collagène.

47. Utilisation d'un conjugué de collagène selon l'une des revendications 19 à 30 pour fabriquer un implant, une prothèse articulaire ou un pansement.

48. Utilisation d'un conjugué de collagène selon l'une des revendications 19 à 30 comme porteur d'affinité pour la purification et l'obtention de protéines liant le collagène.
